(19)

**Europäisches Patentamt**

**European Patent Office**

**Office européen des brevets**

(11) **EP 4 134 450 A1**

(12) **EUROPEAN PATENT APPLICATION**

(43) Date of publication:
**15.02.2023 Bulletin 2023/07**

(51) International Patent Classification (IPC):
*C12Q 1/6883* (2018.01)    *C12Q 1/6886* (2018.01)

(21) Application number: **21382760.3**

(52) Cooperative Patent Classification (CPC):
**C12Q 1/6883; C12Q 1/6886;** C12Q 2600/156

(22) Date of filing: **13.08.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **GENINCODE UK LIMITED**
**Manchester M2 3DE (GB)**

(72) Inventors:
• **SORIA FERNÁNDEZ, José Manuel**
  **08031 Barcelona (ES)**
• **MUÑOZ MARTÍN, Andrés Jesús**
  **28023 Madrid (ES)**
• **CARRERA MARTÍNEZ, Marta**
  **08173 Sant Cugat del Vallés, Barcelona (ES)**
• **WALLS, Matthew**
  **Manchester M2 3DE (GB)**

(74) Representative: **Marks & Clerk LLP**
**15 Fetter Lane**
**London EC4A 1BW (GB)**

Remarks:
The complete document including Reference Table(s) and the Sequence Listing(s) can be downloaded from the EPO website

(54) **PREDICTION OF CANCER-ASSOCIATED VENOUS THROMBOEMBOLISM**

(57)    The present invention concerns the provision of an improved method for the diagnosis and prediction of thrombotic events in cancer patients, and provides additionally respective kits.

EP 4 134 450 A1

**Description**

**FIELD OF THE INVENTION**

The present invention relates to the field of thrombotic diseases or disorders. More specifically, it relates to markers and methods for determining whether a subject, particularly a human subject, suffering or under the suspicion of suffering from cancer, is at risk of developing thromboembolic diseases or disorders, developing a thromboembolic event, having a thromboembolic disease or disorder, or is or will be experiencing a complication of a thromboembolic disease.

TECHNICAL BACKGROUND

Cancer is a major risk factor for venous thromboembolism (VTE), as well as other thromboembolic diseases, and is responsible e.g. for 18% of all cases of incident VTE[1]. Across all patients with cancer, the risk for VTE is elevated 7-fold as compared to non-cancer patients, and in certain malignancies, the risk for VTE may be increased up to 17-fold[2]. VTE is discovered in a fifth of all cancer patients and as many as half of cancer *post-mortem* examinations[3]. The incidence of cancer-associated VTE is particularly high during the first months after diagnosis, if distant metastases are present, and after initiating chemotherapy[2,4,5].

Cancer-associated VTE bears several clinical and economic implications, including increased hospitalization rates and the potential for delays in cancer therapy[6]. In patients with cancer, VTE is the second leading cause of death; in fact, of every seven patients with cancer who die while hospitalised, one will die of pulmonary embolism[1], which is a typical direct consequence of VTE. Even among patients who survive an episode of VTE, complications such as recurrent VTE, post-thrombotic syndrome, and chronic thromboembolic pulmonary hypertension are common, costly, and have a profound impact on the patient's quality of life[1].

Due to the relevance of cancer associated-VTE, scientific societies have already published different guidelines covering the identification of cancer patients at risk, possible prevention strategies and the treatment of cancer-associated VTE[6,7,8,9].

**A) Identification of cancer patients at risk of developing VTE**

Table 1 shows the currently known thrombotic risk factors identified in patients suffering from cancer-associated VTE. Those risk factors can be divided in demographic, cancer related, treatment related, and other risk factors.

Table 1.

| Thrombotic risk factors | | |
|---|---|---|

| Patient | | |
|---|---|---|
| | old age | |
| | ethnicity | |
| | | higher in African Americans |
| | | lower in Asians |
| | Family history of VTE | |
| | Personal history of VTE | |
| | Body mass index | |
| Cancer | | |
| | Site | |
| | | brain |
| | | pancreas |
| | | kidney |
| | | stomach |
| | | lung |
| | | gynaecologic, haematologic malignances |
| | stage | |
| | | advanced stage |
| | | initial period after diagnosis |
| Treatment | | |
| | hospitalization | |
| | surgery | |
| | chemo- and hormonal therapy | |
| | anti-angiogenic therapy | |
| | erythropoiesis stimulating agents | |
| | Megestrol acetate | |
| | central vanous catheter | |
| Other | | |
| | Plaster cast immobilization | |
| | Trauma | |
| | hormonal contraceptive | |
| | pregnancy | |

> collagen vascular diseases
> heart
> failure
> D-dimer
> Nephrotic syndrome
> P-selectin

In the following, additional information is provided on both cancer- and treatment related risk factors.

**Cancer related risk factors**

The so-called "cancer related risk factors" describe the incidence of thrombotic events, diseases or disorder, in particular of VTE, according to tumour type and stage. The rate of e.g. VTE is consistently higher in patients with cancer of the pancreas, stomach, brain, kidney, uterus, lung, prostate, leukemia or ovary[10]. Among the haematological malignancies, lymphoma, leukemia and myeloma disease were reported to have the highest rates of VTE[11,12].

The extent of malignant disease also affects the likelihood of developing thrombotic events, diseases or disorder, in particular of VTE. Multiple studies have shown an increased risk of VTE in patients with advanced-stage cancer[10]. An analysis of data from the California Cancer Registry between 1993 and 1995 highlighted metastatic disease as the strongest predictor of VTE and VTE complications[13]. Similarly, it is known that cancer patients with distant metastases had almost a twofold increased risk of VTE compared with those without metastases.

**Treatment related risk factors**

Many standard anticancer treatment strategies have been shown to increase the risk of thrombotic events, diseases or disorder, in particular of VTE complications. These factors include both surgical procedures and non-surgical treatments. Another important risk factor is the presence of a central venous catheter[14].

Surgery is a well-known risk factor for development of VTE in patients without cancer. Cancer patients undergoing surgery are at increased risk (i. e. 3- to 5-fold) for postoperative thrombosis compared to surgical patients who do not have cancer, and this risk can persist for up to seven weeks after the procedure (Am J Surg 1970; 120: 527-530). VTE is the most common cause of death 30 days after surgery in cancer patients (46%), followed by cancer progression (12%)[15].

Non-surgical anticancer treatment strategies are also associated with a high incidence of thrombotic events, diseases or disorder, in particular of VTE. Active treatments including chemotherapy, adjuvant chemotherapy, hormonal therapy, antiangiogenic agents, and combination regimens all have a prothrombotic effect in cancer patients. Chemotherapy, either as primary or adjuvant therapy, significantly increases the risk of thrombotic events, diseases or disorder, in particular of VTE complications in patients with cancer. Recently, a prospective study of nearly 4500 patients receiving outpatient chemotherapy reported a 2.7-fold increase in arterial thrombosis, and a 47-fold increase in the mortality rate from VTE compared with the general population[16].

Hormone therapy in combination with chemotherapy enhances the incidence of VTE in women with breast cancer. Studies have reported that women who received the selective estrogen receptor modulator tamoxifen had a 1.5- to 7.1-fold increase in the risk of developing symptomatic VTE, compared with placebo or no treatment.

New therapeutic agents that inhibit angiogenesis are being developed as treatments for various solid tumours such as non-small-cell lung cancer, breast cancer, and colon cancer. Targeted antiangiogenic agents, such as bevacizumab, the monoclonal antibody to vascular endothelial growth factor, have shown efficacy in improving survival rates among patients with advanced disease. However, the addition of bevacizumab to chemotherapy regimens is associated with a high incidence of thrombotic events.

The use of recombinant human erythropoietin (EPO) and other haematopoietic growth factors, such as granulocyte-macrophage colony-stimulating factor and granulocyte-colony stimulating factor, as supportive therapy in cancer patients appears to increase the risk of VTE.

Long-term central venous catheters (CVC) have considerably improved the management of cancer patients. However, they also represent a significant risk factor for VTE.

**Risk stratification**

The interaction and relative effects of the risk factors associated with VTE in cancer patients is highly complex, making the pre-treatment assessment of VTE difficult. As summarized in Table 1, several demographic, cancer-associated, and treatment-related factors are known to increase the risk of thrombosis. Until recently, no clinically validated predictive models, including clinical and laboratory markers, have been developed to identify the actual risk of developing thrombotic events, diseases or disorder, in particular of VTE in these patients. However, a VTE risk assessment model for patients undergoing chemotherapy has been published by Khorana et al. based on five predictive variables in cancer patients[17]:

1. cancer site (very high risk: stomach and pancreas; high risk: lung, lymphoma, gynaecologic, bladder, and testicular);
2. pre-chemotherapy platelet count of $\geq 350 \times 109/l$ = high risk;
3. haemoglobin levels < 100 g/l (or the use of erythropoiesis-stimulating agents) = high risk;
4. pre-chemotherapy leukocyte count >11 $\times$ 109/l = high risk;
5. body mass index $\geq 35kg/m^2$ = high risk.

Scientific Societies have published guidelines recommending that patients with cancer be assessed for VTE risk at the time of chemotherapy and periodically thereafter. In outpatient setting, risk assessment can be conducted based on the above validated risk assessment tool developed by Khorana[9,17].

The guidelines with respect to risk stratification schemes for prophylaxis of VTE in patients with cancer are outlined in Farge et al., (2019) *2019 international clinical practice guidelines for the treatment and prophylaxis of venous thromboembolism in patients with cancer,* as summarized below (Panel 4, Farge et al. (2019)).

*Patients with cancer are at increased risk of VTE, which is determined by the clinical setting and the presence of various risk factors. A time-dependent association between VTE and cancer has also been observed, with most VTE events occurring within the first 6 months after cancer diagnosis.*

*Risk factors*

- *Risk factors associated with the tumour characteristics: primary site, histological grade, tumour node metastasis staging*
- *Risk factors associated with the cancer treatments: surgery or hospitalisation; central venous catheters; systemic anticancer therapy, including radiotherapy, chemotherapy (eg, cisplatin), anti-angiogenesis agents, immunomodulatory drugs, hormonal therapy, erythropoiesis-stimulating agents, or red blood cell or platelet transfusions*
- *General individual VTE risk factors: history of previous venous thromboembolism, advanced age, obesity, immobility, prothrombotic variants (eg, factor V Leiden), and comorbidities Emerging biomarkers*
- *Blood-count parameters: platelets and leucocytes*
- *Markers of activation of blood coagulation and platelets: D-dimers, high endogenous thrombin generation potential, soluble P-selectin*
- *Markers of neutrophil extracellular trap formation (ie, citrullinated histone H3)*
- *Microvesicle-associated tissue factor activity*
- *High podoplanin expression and iscocitrate dehydrogenase 1 mutation (in brain tumours only)*

*Risk assessment models*

*The Khorana Risk Scoring Model was developed for VTE risk assessment in patients receiving chemotherapy. This risk score was externally validated in the Vienna CATS study and other studies, although subsequent publications questioned its reproducibility in some patient populations. Several variations of the Khorana risk score have been done to improve risk assessment, including the extended Vienna CATS Score, the PROTECHT, and the CONKO score.*

*The COMPASS-CAT risk assessment model was developed for use in only breast, colorectal, lung, and ovarian cancer and includes the following variables: anthracycline or anti-hormonal therapy, time since cancer diagnosis, central venous catheter, stage of cancer, presence of cardiovascular risk factors, recent hospitalisation for acute medical illness, personal history of venous thromboembolism, and platelet count.*

*The ONKOTEV score is based on a Khorana score of >2, then adds metastatic disease, previous venous thromboembolism, and vascular or lymphatic compression. A combination of genetic and clinical factors was used to develop the TiC-Onco score, which performed better than the Khorana risk score in identifying patients with cancer who are at risk of venous thromboembolism.*

*Pabinger and colleagues followed a prespecified process to develop and externally validate in a single prospective cohort (MICA) of 832 patients with cancer a simple clinical prediction model that only includes the tumour site*

*category (very high and high versus intermediate or low) and D-dimer levels as a continuous variable, and a nomogram and online risk calculator is provided for estimating an individual patient's risk of venous thromboembolism.*

**B) Prophylaxis of thrombotic events, diseases or disorder, in particular of VTE**

The prevention of thrombotic events, diseases or disorder, in particular of VTE in cancer patients is of vital importance in light of the difficulties associated with the treatment of thrombotic events, diseases or disorder, in particular of VTE in these patients, as they are also prone to greater recurrence rates and a higher incidence of bleeding complications[18].

It has been shown that cancer patients undergoing surgery benefit from effective pharmacological prophylaxis, and that extended-duration thromboprophylaxis with low molecular weight heparin (LMWH) may be beneficial to patients undergoing major abdominal or pelvic surgery. This is reflected in the guidelines: "All patients undergoing major cancer surgery should receive heparin-based prophylaxis for a minimum of 7-10 days, with supportive mechanical prophylaxis in those patients at highest risk"[19].

Thromboprophylaxis has been shown to decrease deep venous thrombosis (DVT) specifically in high-risk hospitalized patients.

Several randomized controlled trials of thromboprophylaxis in ambulatory cancer patients have been reported. The most recent trials, conducted in patients with advanced pancreatic cancer who receive systemic chemotherapy, have shown positive results with low molecular weight heparin (LMWH) prophylaxis. In particular, the CONKO-004 trial found 87% risk reduction of VTE using LMWH enoxaparin at 1 mg/kg body weight once daily for three months compared with no prophylaxis (9.9% vs 1.3%; p < 0.01). Recently, LMWH nadroparin was found to reduce the incidence of thromboembolic events in ambulatory cancer patients receiving chemotherapy for metastatic or locally advanced disease.

The recommendations of the various guidelines with respect to prophylaxis of VTE in the different clinical settings are summarized below (Panel 3, Farge et al. (2019)).

*Prophylaxis of VTE in patients with cancer who have undergone surgery*

*International Advisory Panel ranking: 8·63 out of 9*

*1. Use of low-molecular-weight heparin (LMWH) once per day (when creatinine clearance is ≥30 mL/min) or low-dose unfractionated heparin three times per day is recommended to prevent postoperative VTE in patients with cancer; pharmacological prophylaxis should be started 2-12 h preoperatively and continued for at least 7-10 days; there are no data allowing conclusions regarding the superiority of one type of LMWH over another (grade 1A). Values and preferences: LMWH once per day is more convenient.*

*2. There is insufficient evidence to support fondaparinux as an alternative to LMWH for the prophylaxis of postoperative VTE in patients with cancer (grade 2C). Values and preferences: as per the first recommendation above.*

*3. Use of the highest prophylactic dose of LMWH to prevent postoperative VTE in patients with cancer is recommended (grade 1A). Values and preferences: as per the first recommendation above.*

*4. Extended prophylaxis (4 weeks) with LMWH to prevent postoperative VTE after major laparotomy in patients with cancer is indicated in patients with a high VTE risk and low bleeding risk (grade 1A). Values and preferences: longer duration of injections.*

*5. Extended prophylaxis (4 weeks) with LMWH for the prevention of VTE in patients with cancer undergoing laparoscopic surgery is recommended in the same way as for laparotomy (grade 2C). Values and preferences: daily injections. Costs: in some countries, the price of LMWH might influence the choice.*

*6. Mechanical methods are not recommended as monotherapy except when pharmacological methods are contraindicated (grade 2B). Values and preferences: no injection.*

*7. Inferior vena cava filters are not recommended for routine prophylaxis (grade 1A).*

*Prophylaxis of VTE in patients with medical cancer*

*International Advisory Panel ranking: 8·00 out of 9*

*1. We recommend prophylaxis with LMWH or fondaparinux when creatinine clearance is ≥30 mL/min, or with unfractionated heparin in hospitalised patients with cancer and reduced mobility (grade 1B). In this setting, direct oral*

*anticoagulants are not recommended routinely (guidance). Values and preferences: subcutaneous injections. Costs: In some countries price differences between LMWH, unfractionated heparin, or fondaparinux might influence the choice.*

*2. Primary prophylaxis with LMWH, vitamin K antagonists, or direct oral anticoagulants in ambulatory patients receiving systemic anticancer therapy is not recommended routinely (grade 1B). Values and preferences: subcutaneous injections.*

*3. Primary pharmacological prophylaxis of VTE with LMWH is indicated in ambulatory patients with locally advanced or metastatic pancreatic cancer treated with systemic anticancer therapy and who have a low risk of bleeding (grade 1B). Values and preferences: subcutaneous injections.*

*4. Primary pharmacological prophylaxis of VTE with LMWH is not recommended outside of a clinical trial for patients with locally advanced or metastatic lung cancer treated with systemic anticancer therapy, including patients who have a low risk of bleeding (guidance).*

*5. Primary prophylaxis with direct oral anticoagulant (rivaroxaban or apixaban) is recommended in patients who are ambulatory who are receiving systemic anticancer therapy at intermediate-to-high risk of VTE, identified by cancer type (ie, pancreatic) or by a validated risk assessment model (ie, a Khorana score ≥2), and not actively bleeding or not at a high risk of bleeding (grade 1B).*

*6. In patients treated with immunomodulatory drugs combined with steroids or other systemic anticancer therapies, VTE primary pharmacological prophylaxis is recommended (grade 1A); in this setting, vitamin K antagonists at low or therapeutic doses, LMWH at prophylactic doses, and low-dose aspirin can be used and have shown similar effects with regard to preventing VTE (grade 2C). Values and preferences: subcutaneous injections.*

*Prophylaxis of catheter-related thrombosis*

*International Advisory Panel ranking: 8·51 out of 9*

*1. Use of anticoagulation for routine prophylaxis of catheter-related thrombosis is not recommended (grade 1A). Values and preferences: bleeding risk with anticoagulants.*

*2. Catheters should be inserted on the right side, in the jugular vein, and the distal extremity of the central catheter should be located at the junction of the superior vena cava and the right atrium (grade 1B).*

*3. In patients requiring central venous catheters, we suggest the use of implanted ports over peripherally inserted central catheter lines (guidance).*

**C) Treatment of thrombotic events, diseases or disorder, in particular of VTE**

Patients with cancer who develop thrombotic events, diseases or disorder, in particular a VTE episode should be managed according to the guidelines. Except for selected patients requiring aggressive treatments, the large majority of cancer patients who develop thromboembolic events, diseases or disorder, in particular a VTE episode, should be treated with therapeutic doses of unfractionated heparin (UFH) or low-molecular-weight heparin (LMWH), followed by LMWH as the preferred agent for long-term monotherapy.

Novel oral anticoagulants have emerged that can rapidly change the therapeutic scenario in patients with/without cancer. These oral anticoagulants that achieve rapid inhibition of activated factor X or thrombin may offer an easier solution than LMWH but studies focusing on treatment of cancer-associated thrombosis with these agents are lacking. To date, some of these new agents have shown comparable efficacy and safety compared with traditional anticoagulants in randomized trials that included primarily patients without cancer.

One problem with this treatment is that there is no possibility to determine for how long the treatment for the VTE should be given.

The guidelines with respect to treatment of established VTE are summarized below (Panel 2, Farge et al. (2019).

*Initial treatment of established VTE*

*International Advisory Panel ranking: 8·18 out of 9*

*1. Low-molecular-weight heparin (LMWH) is recommended for the initial treatment of established VTE in patients with cancer when creatinine clearance is ≥30 mL per min (grade 1B). Values and preferences: LMWH is easier to*

use than unfractionated heparin. A regimen of LMWH, taken once per day, is recommended, unless a twice-per-day regimen is required because of patient characteristics (eg, fragile patients who are at risk of haemorrhage).

2. For patients who do not have a high risk of gastrointestinal or genitourinary bleeding, a regimen of rivaroxaban (in the first 10 days) or edoxaban (started after at least 5 days of parenteral anticoagulation) can also be used for the initial treatment of established VTE in patients with cancer when creatinine clearance is ≥30 mL/min (grade 1B).

3. Unfractionated heparin can also be used for the initial treatment of established VTE in patients with cancer when LMWH or direct oral anticoagulants are contraindicated, or not available (grade 2C).

4. Fondaparinux can also be used for the initial treatment of established VTE for patients with cancer (grade 2D). Values and preferences: fondaparinux is easier to use than unfractionated heparin.

5. Thrombolysis in patients with cancer with established VTE can only be considered on a case-by-case basis, with specific attention paid to contraindications, especially bleeding risk-eg, brain metastasis (guidance, based on evidence of very low quality and the high bleeding risk of thrombolytic therapy). Values and preferences: an expert opinion is recommended before using thrombolytics, and the procedure should be done in centres with health-care practitioners who have appropriate expertise.

6. In the initial treatment of VTE, inferior vena cava filters may be considered when anticoagulant treatment is contraindicated or, in the case of pulmonary embolism, when recurrence occurs under optimal anticoagulation. Periodic reassessment of contraindications for anticoagulation is recommended, and anticoagulation should be resumed when safe (guidance, based on evidence of very low quality and an unknown balance between desirable and undesirable effects).

Early maintenance (up to 6 months) and long term (beyond 6 months)

International Advisory Panel ranking: 8·09 out of 9

1. LMWHs are preferred over vitamin K antagonists for the treatment of VTE in patients with cancer when creatinine clearance is ≥30 mL/min (grade 1A). Values and preferences: daily subcutaneous injection can represent a burden for patients.

2. Direct oral anticoagulants are recommended for patients with cancer when creatinine clearance is ≥30 mL/min in the absence of strong drug-drug interactions or gastrointestinal absorption impairment (grade 1A). Use caution in patients with gastrointestinal tract malignancies, especially upper gastrointestinal tract malignancies, as the available data show increased risk of gastrointestinal tract bleeding with edoxaban and rivaroxaban. Data for other direct oral anticoagulants are needed as it is not clear whether other direct oral anticoagulants will have the same risk profile.

3. LMWH or direct oral anticoagulants should be used for a minimum of 6 months to treat established VTE in patients with cancer (grade 1A).

4. After 6 months, termination or continuation of anticoagulation (LMWH, direct oral anticoagulants, or vitamin K antagonists) should be based on individual evaluation of the benefit risk ratio, tolerability, drug availability, patient preference, and cancer activity (guidance in the absence of data).

Treatment of VTE recurrence in patients with cancer under anticoagulation

International Advisory Panel ranking: 8·0 out of 9

In the event of VTE recurrence, three options can be considered: (1) increase LMWH by 20—25% or switch to direct oralanticoagulants; (2) for direct oral anticoagulants, switch to LMWH; and (3) for vitamin K antagonists, switch to LMWH or direct oral anticoagulants (guidance based on evidence of very low quality and an unknown balance between desirable and undesirable effects). Values and preferences: individual decision. Effect of therapy should be monitored by improvement of symptoms.

Treatment of established catheter-related thrombosis

International Advisory Panel ranking: 8·19 out of 9

1. For the treatment of symptomatic catheter-related thrombosis in patients with cancer, anticoagulant treatment is recommended for a minimum of 3 months and as long as the central venous catheter is in place; in this setting,

*LMWHs are suggested and direct comparisons between LMWHs, direct oral anticoagulants, and vitamin K antagonists have not been made (guidance).*

*2. In patients with cancer with catheter-related thrombosis, the central venous catheter can be kept in place if it is functional, well positioned, and not infected, with a good resolution of symptoms under close surveillance while anticoagulation therapy is administered. No standard approach in terms of duration of anticoagulation is established (guidance).*

**D) Identification of cancer patients at risk of developing recurrent thrombotic events, diseases or disorder, in particular of VTE**

Cancer patients are at high risk for recurrent thrombotic events, diseases or disorder, in particular of VTE. In a prospective cohort study, 20.7% of cancer patients developed recurrent VTE compared with 6.8% of patients without cancer. Cancer patients experience recurrence even within the first six month of anticoagulation. However, this risk varies depending on several parameters such as cancer site, cancer histology, or cancer stage.

There are few scores to predict VTE recurrence and only one is applicable for cancer patients. However, none of these scores for VTE recurrence prediction has been properly evaluated and none of them is used worldwide in clinical practice.

NEED FOR A NEW RISK PREDICTION SCORE IN CANCER PATIENTS FOR THROMBOTIC EVENTS, DISORDERS OR DISEASES, AND A NEW RECURRENCE RISK PREDICTION SCORE FOR THROMBOTIC EVENTS, DISORDERS OR DISEASES IN CANCER PATIENTS. Despite all the research done in the last years, the predictive capacity to identify cancer patients at risk of developing a first or recurrent thrombotic event, disorder or disease, in particular VTE, using the nowadays reference scores is very low. The performance of the Khorana score as described above is as follows: The risk model was tested in a validation cohort of 1365 patients of whom 28 (2.1%) developed VTE. At the cut-off point for high risk (score 3), the model had a negative predictive value (probability of no VTE in those designated low risk) of 98.5%, a very low positive predictive value (probability of VTE in those designated high risk) of 7.1%, a low sensitivity (probability of high risk in those experiencing VTE) of 40.0%, and a specificity (probability of low risk in those not experiencing VTE) of 88% in the derivation cohort. Similarly, in the validation cohort, the model had a negative predictive value of 98.5%, a very low positive predictive value of 6.7%, a low sensitivity of 35.7%, and a specificity of 89.6%. Moreover, in the last years several studies have shown that the application of Khorana is not enough as the unique tool to identify cancer patients who should receive thromboprophylaxis[20—22].

Scores to predict VTE recurrence have not been properly analysed regarding sensitivity, specificity and predictive values. From the data published so far, it can be deducted that the sensitivity thereof is 24.9%, which would be extremely low.

Accordingly, there is a need for novel markers, including new genetic markers and clinic variables, and specific combinations thereof, that would successfully and advantageously predict which cancer patients are at high risk of developing a recurrence of thromboembolic disease and/or thromboembolic disease complications such as - but not limited to - deep vein thrombosis and/or pulmonary embolism, whereby such a prediction would make it possible that preventive measures could be implemented as soon as possible to keep that risk at the lowest possible level.

WO2018215590A1 teaches a method for the thromboembolic event risk assessment in a subject suffering from cancer. This method has been demonstrated to be significantly better than the standard/gold standard method: the Khorana score.

**SUMMARY OF THE INVENTION**

We have recently identified a new series of genetic variants which are associated with a particularly high risk of presenting a thromboembolic event and/or recurrent thromboembolic event. This new series of genetic variants shows improved predictive and diagnostic value beyond both the Khorana score (as shown in the examples below), and the methods of WO2018215590A1 (Table 13), and are the basis of the present disclosure.

According to the present disclosure, there are provided methods which are suitable for improving the results described in WO2018215590A1 CANCER-ASSOCIATED VENOUS THROMBOEMBOLIC EVENTS and for overcoming the limitations of the methods used nowadays to estimate the thromboembolism risk and/or to diagnose the thromboembolic events for a subject suffering from cancer.

According to a first aspect of the present invention, there is provided a method for thromboembolic event risk assessment in a subject suffering from cancer comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of the risk of suffering a thromboembolic disease or event and/or a recurrent thromboembolic disease or event.

According to a second aspect of the present invention, there is provided a method for the diagnosis of developing or suffering a thromboembolic disease or event or a recurrent thromboembolic event in a subject suffering from cancer comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of the risk of suffering a thromboembolic disease or event and/or a recurrent thromboembolic disease or event.

In embodiments, methods may further comprise the steps of determining in the sample the presence or absence of at least one, two, three, four or preferably all of the following polymorphisms: rs6025, rs4524, rs2227631, rs169713, and rs5110, or respective SNPs in strong linkage disequilibrium with these variants, whereby said presence is indicative of the risk of suffering a thromboembolic disease or event and/or a recurrent thromboembolic disease or event.

In embodiments, the thromboembolic disease or event is selected from the group of fatal or non-fatal myocardial infarction, stroke, transient, ischemic attacks, peripheral arterial disease, vein thrombosis, deep vein thrombosis, pulmonary embolism or a combination thereof, preferably from venous thromboembolism, deep vein thrombosis and pulmonary embolism.

According to a third aspect of the present invention, there is provided a method for identifying a subject suffering from cancer in need of anticoagulant and/or antithrombotic therapy or in need of prophylactic antithrombotic and/or anticoagulant therapy comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of being in need of early and aggressive antithrombotic and/or anticoagulant therapy or in need of prophylactic antithrombotic and/or anticoagulant therapy.

In embodiments, methods may further comprise the steps of determining in the sample the presence or absence of at least one, two, three, four, or preferably all of the following polymorphisms: rs6025, rs4524, rs2227631, rs169713, and rs5110, or respective SNPs in strong linkage disequilibrium with these variants, whereby said presence is indicative of being in need of early and aggressive antithrombotic and/or anticoagulant therapy or in need of prophylactic antithrombotic and/or anticoagulant therapy.

In embodiments, methods may further comprise determining one or more risk factor(s) selected from the group consisting of body mass index (BMI), primary site of the tumor and tumor stage, optionally wherein each of body mass index (BMI), primary site of the tumor, and tumor stage are determined.

In embodiments, methods may be performed during a procedure to identify whether the subject is suffering from cancer and/or during a procedure to characterize the TNM stage of the cancer.

In embodiments, methods may be performed at any time from the diagnosis of the cancer.

In embodiments, the subject suffering from cancer may be treated in an 'in or out-patient' setting.

In embodiments, the sample is an oral tissue sample, scraping or wash or a biological fluid sample, preferably saliva, urine or blood, or buccal cells.

In embodiments, the presence or absence of the genetic variants is identified by allele-specific PCR or other molecular identification methods.

According to a fourth aspect of the present invention, there is provided a method of determining the probability of an individual suffering from cancer of presenting a thromboembolic disease or event or a recurrent thromboembolic disease or event, based on the presence of 1 to P classical risk factors and 1 to J polymorphisms selected from the group of rs2232698, rs6025, rs4524, rs2227631, rs268, rs169713, rs11696364, rs5110, and/or rs6003, or respective SNPs in strong linkage disequilibrium with these variants, using the formula (function 1):

$$\text{Probability } (Y=1|X_1, ..., X_n) = 1/ 1 + \exp (\beta_o + \beta_1 X_1 + ... + \beta_n X_n + \beta_{f \cdot g}\, x_f \cdot X_g + .... \beta_{h \cdot i}\, x_h \cdot x_i),$$

wherein:

- Probability $(Y = 1 | X_1, ..., X_n)$ = probability of presenting a thrombosis in a particular cancer patient - with concrete and measurable characteristics in a number of variables 1, ...., n;
- Exp = exponential natural base;
- $\beta_0$ = coefficient that defines the risk (the probability) of thrombosis non related with the variables 1 to n;
- $\beta_1$ = regression coefficient that expresses the risk (higher or lower) to present thrombosis associated with the value/presence of the predictor variable X1;
- $X_1$ = value taken by the predictor variable X1 in an individual;
- $\beta_n$ = regression coefficient that expresses the risk (higher or lower) to present thrombosis associated with the value/presence of the predictor variable $X_n$;
- $X_n$ = value taken by the predictor variable $X_n$ in an individual;

and

- $\beta_{f \cdot g}$ = regression coefficient that expresses the risk (higher or lower) to present thrombosis associated with the combined presence of the predictor variables $X_f$ and $X_g$;
- $X_f$ = value taken by the predictor variable $X_f$ in an individual;
- $X_g$ = value taken by the predictor variable $X_g$ in an individual;
- $\beta_{h \cdot i}$ = regression coefficient that expresses the risk (higher or lower) to present thrombosis associated with the combined presence of the predictor variables $X_h$ and $X_i$;
- $X_h$ = value taken by the predictor varible $X_h$ in an individual; and
- $X_i$ = value taken by the predictor variable $X_i$ in an individual.

14. The method of claim 13, wherein the 1 to J polymorphisms comprise rs2232698, rs268, rs11696364, and rs6003, namely Serpin A10 (protein Z inhibitor) Arg67Stop (rs2232698), LDL (rs268), RSPO4 (rs11696364), and factor 13 (rs6003).

In embodiments, the 1 to J polymorphisms comprise rs2232698, rs268, rs11696364, and rs6003, or respective SNPs in strong linkage disequilibrium with these variants; and preferably wherein the 1 to J polymorphisms further comprise at least one, two, three, four, or preferably all of rs6025, rs4524, rs2227631, rs169713, and rs5110, or respective SNPs in strong linkage disequilibrium with these variants.

In embodiments, the 1 to P classical risk factors are selected from the group consisting of BMI, primary site of the tumor and tumor stage, optionally wherein the 1 to P classical risk factors include each of BMI, primary site of the tumor and tumor stage.

In embodiments, the detection of the presence or absence of the polymorphisms may be carried out at a separate location from the subject, and optionally the risk assessment or the need for therapy may be reported back to the subject and/or a clinician from that separate location.

In embodiments, methods may further comprise the steps of a) receiving, at a location separate from that of the subject, the sample isolated from the subject; and b) sending the results of the method from said separate location to the subject and/or a clinician.

According to a fifth aspect of the present invention, there is provided a computer program or a computer-readable media containing means for carrying out the method of any one preceding claim.

According to a sixth aspect of the present invention, there is provided a kit comprising reagents for detecting the identity of the nucleotides from the group of rs2232698, rs268, rs11696364, and rs6003, or respective SNPs in strong linkage disequilibrium with these variants.

In embodiments, the kit may further comprise reagents for detecting the identity of the nucleotides from at least one, two, three, four, or preferably all of rs6025, rs4524, rs2227631, rs169713, and rs5110, or respective SNPs in strong linkage disequilibrium with these variants.

In embodiments, the reagents may comprise primer pairs specific for the amplification of regions comprising the variants.

In embodiments, a strong linkage disequilibrium may be one with an $r^2$ value of more than 0.7, more than 0.8, or more than 0.9, wherein $r^2$ is defined as follows:

$$r^2(p_a, p_b, p_{ab}) = \frac{(p_{ab} - p_a p_b)^2}{p_a(1 - p_a) p_b(1 - p_b)},$$

where $p_{ab}$ is the frequency of haplotypes having allele $a$ at locus 1 and allele $b$ at locus 2

In embodiments, the respective SNPs in strong linkage disequilibrium with the variants may be selected from the SNPs in Table 3.

In another aspect, the invention relates to methods for establishing the probability of a patient suffering from cancer of presenting a thromboembolic event based on the presence of one or more of the polymorphisms mentioned above, optionally in combination with one or more predictive variables, e.g. as described above.

In another aspect, the invention relates to methods for establishing the probability of a patient suffering from cancer of presenting a recurrent thromboembolic event based on the presence of one or more of the polymorphisms mentioned above, optionally in combination with one or more predictive variables, e.g. as described above.

In another aspect, the invention relates to methods for the assistance in the diagnosis in a patient suffering from cancer, of a thromboembolic event, based on the presence of one or more of the genetic variants mentioned above, optionally in combination with one or more predictive variables, e.g. as described above, optionally, to confirm an earlier tentative diagnosis of a thromboembolic event.

In another aspect, the invention relates to methods for determining the need for preventive measurements in a patient suffering from cancer to prevent the development of a thromboembolic event and/or a recurrent thromboembolic event based on the presence of one or more of the polymorphisms mentioned above, optionally in combination with one or more predictive variables, e.g. as described above.

In another aspect, the invention relates to methods for the determination of a need for the prolonged treatment with anticoagulants in a patient suffering from cancer to prevent the development of a recurrent thromboembolic event based on the presence of one or more of the polymorphisms mentioned above, optionally in combination with one or more predictive variables, e.g. as described above.

In a further aspect, the invention relates to a computer program or a computer-readable media containing means for carrying out any of the methods of the invention, in particular means which allow for the determination of the above preferred selection of genetic variants, and/or means for the determination of the above preferred additional variables.

In a further preferred embodiment, the kit comprises those reagents which are suitable for detecting the preferred or more preferred combination of genetic variants, as listed above.

In an even further preferred embodiment, the kit comprises reagents which together with the additional clinical variables, enables an assessment of the VTE risk.

The person of skill in art, when informed about the preferred selection of SNPs and e.g. further variables, is well aware of methods and means on how to determine these variables. E.g. it is within the usual general knowledge of the person skilled in the art to determine probes or primers suitable to detect the above genetic variables. Also, it is well within the ambit of an average person of skill in the art to determine the additional variables, like BMI.

## DETAILED DESCRIPTION OF THE INVENTION

The authors of the present invention have solved the problems identified above in the methods in use nowadays for the calculation of the risk in a subject to develop a thromboembolic event and/or recurrent thromboembolic event, as those terms have been defined above.

The authors of the present invention have identified a series of genetic variants which are associated with a particularly high risk of presenting a thromboembolic event and/or a recurrent thromboembolic event. These genetic variants show improved predictive and diagnostic value.

The present application solves the above-described limitations of the methods used nowadays to calculate a thromboembolic event risk and/or a recurrent thromboembolic event risk and/or to diagnose a thromboembolic event, in particular of VTE, and in particular in cancer patients. This is achieved if a particular combination (as described above) of genetic markers is used, which has been selected and evaluated by the inventors after a complex and genuine analysis of thousands of possible markers. Of the different possibilities to construct a genetic risk score (GRS), the inventors have determined a particular preferred combination and show, as proven in the examples below, that it provides the best possible results, results that have been also validated in another population. To calculate the genetic risk or GRS, the accumulated number of risk alleles from those SNPs listed in table 2 below that are present in each individual is considered.

Table 2

| Variable | Beta value |
|---|---|
| GRS | 0.95217 |
| rs4524 | 0.46909 |
| rs6025 | 0.46277 |
| rs2232698 | 1.20232 |
| rs2227631 | -0.32215 |
| rs268 | 0.88709 |
| rs169713 | -0.48158 |
| rs11696364 | 0.82290 |
| rs5110 | 0.50068 |
| rs6003 | 0.62737 |
| "GRS" (genetic risk score) here refers to the GRS for the nine preferred SNPs. | |

As shown in Table 2, beta values for rs2232698, rs268, rs11696364, and rs6003 are particularly high, indicating a strong relationship between the presence of these SNPs and thromboembolic risk. In one example, a genetic risk score may therefore be calculated based on the presence or absence of these four SNPs.

For any given SNP, as mentioned herein, also all SNPs in high linkage disequilibrium are encompassed.

"Linkage disequilibrium" is well known to the person skilled in the art who will understand how to determine further SNPs in linkage disequilibrium to a given SNP.

A strong linkage disequilibrium is one with an $r^2$ value of more than 0.7, 0.8, or 0.9, wherein $r^2$ is defined as follows:

$$r^2(p_a,p_b,p_{ab})=\frac{(p_{ab}-p_ap_b)^2}{p_a(1-p_a)p_b(1-p_b)},$$

where $p_{ab}$ is the frequency of haplotypes having allele $a$ at locus 1 and allele $b$ at locus 2

The respective information can also be acquired in a straight-forward manner from public genome browsers like http://www.ensembl.org.

From such web resources, the skilled person can easily retrieve variants that are in linkage disequilibrium with a certain SNP.

Examples of SNPs in strong linkage disequilibrium with the variants rs4524, rs169713, rs268, and rs6003, are shown in Table 3 below.

Table 3

| Original SNP | SNPs in strong LD | $R^2$ |
|---|---|---|
| rs4524 - F5 K858R | rs12127364 | 0.963499 |
|  | rs10800450 | 0.963499 |
|  | rs4656684 | 0.963499 |
|  | rs2009814 | 0.963499 |
| rs169713 - cr. 6 | rs209773 | 0.942562 |
|  | rs209774 | 0.989267 |
|  | rs209776 | 0.952651 |
|  | rs209777 | 0.85846 |
| rs268 - LPL | rs190118115 | 0.932392 |
|  | rs75835816 | 0.860374 |
|  | rs34356624 | 0.860374 |
| rs6003 - F13B | rs6702340 | 0.926521 |
|  | rs6694672 | 0.926521 |
|  | rs6657171 | 0.926521 |
|  | rs6657246 | 0.926521 |

In an example, SNPs in high linkage disequilibrium may be those with more than 70%, more preferred more than 80%, even more preferred more than 95% linkage disequilibrium.

For each of the genetic variants studied, every individual can have 0, 1 or 2 alleles of risk. On having calculated the summary of weighted risk alleles accumulated in the different set of the selected variants (n=9), for each individual a score that could go from 0 to 18 was given. A higher score means a higher risk. In an example, to establish the score, it is possible to use the beta values for each genetic variable (see Table 2). You can have 0, 1 or 2 times (depending on the number of risk alleles found in a given sample, per SNP) that beta value per each variant and the sum will give the weighted genetic score.

In a further preferred embodiment, the above genetic variants are combined with additional predictive variables (see Table 4). Per each of the further predictive variables, "0" indicaties the absence of this predictive variable and "1" indicates its presence. However, for the determination of "stage", for TNM I and II the value of 0 is given, for TNM II and III a value of 1 is given, and for TNM IV a value of 2 is given.

Table 4

| Variable | Beta value |
|---|---|
| BMI>25 | 0.45852 |

(continued)

| Variable | Beta value |
|---|---|
| TumorTypeKhHR | -0.36212 |
| TumorTypeKhVHR | 0.77233 |
| Stage | 1.27356 |

"Stage" is the stage of the TNM tumor staging system described above. This staging system is well accepted and can be used for the present tumor stage definition.

Higher tumor stages mean a higher risk (as described in e.g. https://www.cancer.gov/about-cancer/diagnosis-staging/staging. "T" refers to the size and extent of the main tumor, also called the primary tumor; "N" refers to the number of nearby lymph nodes that have cancer; "M" refers to whether the cancer has metastasized, which means that the cancer has spread from the primary tumor to other parts of the body).

The TNM tumor staging can be further categorized as follows:

Primary tumor (T)

- TX - main tumor cannot be measured
- T0 - main tumor cannot be found
- T1, T2, T3, T4 - refers to the size and/or extent of the main tumor. The higher the number after the T, the larger the tumor or the more it has grown into nearby tissues.

Regional lymph nodes (N)

- NX - cancer in nearby lymph nodes cannot be measured
- N0 - there is no cancer in nearby lymph nodes
- N1, N2, N3 - refers to the number and location of lymph nodes that contain cancer. The higher the number after the N, the more lymph nodes that contain cancer.

Distant metastasis (M)

- MX - metastasis cannot be measured
- M0 - cancer has not spread to other parts of the body
- M1 - cancer has spread to other parts of the body.

A further predictive variable included in the score is primary site of the tumor, e.g. as determined in the Khorana score. "KhHR" means high risk based on the Khorana score (i.e. +1), and "KhVHR" means very high risk based on the Khorana Score (i.e. +2). (Very high risk: stomach, pancreas; high risk: lung, lymphoma, gynecologic, genitourinary excluding prostate; low risk: breast, colorectal, head and neck).

The sum of the genetic variants and further predictive variables, preferably using the function 1 described below, will result in the clinic-genetic overall score. Therefore, the inventors have additionally generated new algorithms for the thromboembolic risk estimation which further assist the determination of a risk and the actual diagnosis, which in turn will lead to an indication to the person of skill in the art of whether or not treatment or preventive measures should be taken to prevent and/or treat a thromboembolic event, in particular VTE. When prediction models are used, as for instance, for making treatment decisions, predictive risks may be categorized by using risk cut-off threshholds.

Those skilled in the art will readily recognize that the analysis of the nucleotides present according to the method of the invention in an individual's nucleic acid can be done by any method or technique capable of determining nucleotides present in a polymorphic site. As it is obvious in the art, the nucleotides present in the polymorphic markers can be determined from either nucleic acid strand or from both strands.

Once a biological sample from a subject has been obtained (e.g., a bodily fluid, such as urine, saliva, plasma, serum, or a tissue sample, such as a buccal tissue sample or a buccal cell, preferably a buccal cell), most preferably blood (plasma or serum), saliva or buccal cells, the detection of sequence variations or allelic variants in the SNPs is typically undertaken. Virtually any method from a variety of methods that exist for detecting sequence variations known to the skilled artisan can be employed and used in the methods of the invention. The particular method used is not important.

One of the most direct and well known methods is to actually determine the sequence of either genomic DNA or cDNA and compare these sequences to the known alleles in those SNPs. This can be a fairly expensive and time consuming process. Nevertheless, this technology is quite common and is well-known to the average person of skill in the art. Other examples exist of possible commercially available methods for high throughput SNP identification not using direct sequencing technologies, for example, Illumina's Veracode Technology, Taqman® SNP Genotyping Chemistry and KASPar SNP genotyping Chemistry.

A variation on the direct sequence determination method is the Gene Chimp™ method available from Affymetrix®. Alternatively, robust and less expensive ways of detecting DNA sequence variation are also commercially available. For example, Perkin Elmer adapted its TAQman Assay™ to detect sequence variation. Orchid BioSciences has a method called SNP-IT™ (SNP-Identification technology) that uses primer extension with labeled nucleotide analogs to determine which nucleotide occurs at the position immediately 3' of an oligonucleotide probe, the extended base is then identified using direct fluorescence, indirect colorimetric assay, mass spectrometry, or fluorescence polarization. Sequenom® uses a hybridization capture technology plus MALDI-TOF (Matrix Assisted Laser Desorption/ionization-Time-of Flight mass spectrometry) to detect SNP genotypes with their MassARRAY™ system. Promega® provides the READIT™ SNP/Genotyping System (U.S. Pat. No 6,159,693). In this method, DNA or RNA probes are hybridized to target nucleic acid sequences. Probes that are complementary to the target sequence at each base are depolymerized with a proprietary mixture of enzymes, while probes which differ from the target at the interrogation position remain intact. The method uses pyrophosphorylation chemistry in combination with luciferase detection to provide a highly sensitive and adaptable SNP scoring system. Third Wave Technologies® has the Invader OS™ method that uses proprietary Cleavage enzymes, which recognize and cut only the specific structure formed during the Invader process. Invader OS® relies on linear amplification of the signal generated by the Invader process, rather than on the exponential amplification of the target. The Invader OS assay does not utilize PCR in any part of the assay. In addition, there are a number of forensic DNA testing labs and many research labs that use gene-specific PCR, followed by restriction endonuclease digestion and gel electrophoresis (or other size separation technology) to detect restriction fragment length polymorphisms (RFLPs).

In various embodiments of any of the above aspects, the presence or absence of the SNPs is identified by amplifying or failing to amplify an amplification product from the sample. Polynucleotide amplifications are typically template-dependent. Such amplifications generally rely on the existence of a template strand to make additional copies of the template. Primers are short nucleic acids that are capable of priming the synthesis of a nascent nucleic acid in a template-dependent process, which hybridize to the template strand. Typically, primers are from ten to thirty base pairs in length, but longer sequences can be employed. Primers may be provided in double-stranded and/or single-stranded form, although the single-stranded form generally is preferred. Often, pairs of primers are designed to selectively hybridize to distinct regions of a template nucleic acid and are contacted with the template DNA under conditions that permit selective hybridization. Depending upon the desired application, high stringency hybridization conditions may be selected that will only allow hybridization to sequences that are completely complementary to the primers. In other embodiments, hybridization may occur under reduced stringency to allow for amplification of nucleic acids containing one or more mismatches with the primer sequences. Once hybridized, the template-primer complex is contacted with one or more enzymes that facilitate template-dependent nucleic acid synthesis. Multiple rounds of amplification, also referred to as "cycles," are conducted until a sufficient amount of amplification product is produced.

Polymerase Chain Reaction

A number of template dependent processes are available to amplify the oligonucleotide sequences present in a given template sample. One of the best known amplification methods is the polymerase chain reaction. In PCR, pairs of primers that selectively hybridize to nucleic acids are used under conditions that permit selective hybridization. The term "primer", as used herein, encompasses any nucleic acid that is capable of priming the synthesis of a nascent nucleic acid in a template-dependent process. Primers may be provided in double-stranded or single-stranded form, although the single-stranded form is preferred. Primers are used in any one of a number of template dependent processes to amplify the target gene sequences present in a given template sample. One of the best known amplification methods is PCR, which is described in detail in U.S. Pat. Nos. 4,683,195, 4,683,202 and 4,800,156, each incorporated herein by reference. In PCR, two primer sequences are prepared which are complementary to regions on opposite complementary strands of the target gene(s) sequence. The primers will hybridize to form a nucleic acid primer complete if the target-gene(s) sequence is present in a sample. An excess of deoxribonucleoside triphosphates is added to a reaction mixture along with a DNA polymerase, e.g. Taq polymerase that facilitates template-dependent nucleic acid synthesis. If the target-gene(s) sequence primer complex has been formed, the polymerase will cause the primers to be extended along the target-gene(s) sequence by adding on nucleotides. By raising and lowering the temperature of the reaction mixture, the extended primers will dissociate from the target-gene(s) to form reaction products, excess primers will bind to the target-gene(s) and to the reaction products and the process is repeated. These multiple rounds of amplification, referred to as "cycles" are conducted until a sufficient amount of amplification product is produced.

In an example, the presence or absence of the genetic variants is identified by allele-specific PCR. This allows for discrimination between the two alleles of a genetic variant. Suitable methods for allele-specific PCR are known to the person skilled in the art. For example, a genotyping assay may comprise PCR primer pairs and probes for allelic discrimination (e.g. with fluorescent reporter dyes). At the end of the PCR reaction, the fluorescent signal for the reporter dyes is measured. The ratio of the signals will be indicative for the genotype of the sample. Alternatively, a common reverse primer may be used with allele-specific forward primers with different tails, and the amplified product used to determine which allele(s) are present in the sample.

The amplification product may be digested with a restriction enzyme before analysis. In still other embodiments of any of the above aspects, the presence or absence of the SNP is identified by hybridizing the nucleic acid sample with primer labeled with a detectable moiety. In other embodiments of any of the above aspects the detectable moiety is detected in an enzymatic assay, radio assay, immunoassay or by detecting fluorescence. In other embodiments of any of the above aspects, the primer is labeled with a detectable dye (e.g., SYBR Green 1, YO-PRO-1, thiazole orange, Hex, pico green, edans, fluorescein, FAM, or TET). In other embodiments of any of the above-aspects, the primers are located on a chip. In other embodiments of any of the above aspects, the primers for amplification are specific for said SNPs.

Another method for amplification is the ligase chain reaction ("LCR"). LCR differs from PCR because it amplifies the probe molecule rather than producing an amplicon through polymerization of nucleotides. In LCR, two complementary probe pairs are prepared and in the presence of a target sequence, each pair will bind to opposite complementary strands of the target such that they abut. In the presence of a ligase, the two probe pairs willing to form a single unit. By temperature cycling, as in PCR, bound ligated units dissociate from the target and then serve as "target sequences" for ligation of excess probe pairs. U.S. Pat. No. 4,883,750, incorporated herein by reference, describes a method similar to LCR for binding probe pairs to a target sequence.

Isothermal Amplification

As isothermal amplification method, in which restriction endonucleases and ligases are used to achieve the amplification of target molecules that contain nucleotide 5'-[[alpha]-thio}-triphosphates in one strand of a restriction site also may be useful in the amplification of nucleic acids in the present invention. In one embodiment, loop-mediated isothermal amplification (LAMP) method is used for single nucleotide polymorphism (SNP) typing.

Strand Displacement Amplification

Strand Displacement Amplification (SDA) is another method of carrying out isothermal amplification of nucleic acids which involves multiple rounds of strand displacement and synthesis, i.e., nick translation. A similar method, called Repair Chain Reaction (RCR), involves annealing several probes throughout a region targeted for amplification, followed by a repair reaction in which only two of the four bases are present. The other two bases can be added as biotinylated derivatives for easy detection.

Transcription-Based Amplification

Other nucleic acid amplification procedures include transcription-based amplification systems, including nucleic acid sequence based amplification. In nucleic acid sequence based amplification, the nucleic acids are prepared for amplification by standard phenol/chloroform extraction, heat denaturation of a clinical sample, treatment with lysis buffer and mini-spin columns for isolation of DNA and RNA or guanidinium chloride extraction of RNA. These amplification techniques involve annealing a primer, which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by a polymerase such as T7 or SP6. In an isothermal cyclic reaction, the RNA's are revers transcribed into double stranded DNA, and transcribed once against with a polymerase such as T7 or SP6. The resulting products, whether truncated or complete, indicate target specific sequences.

Other amplification methods may be used in accordance with the present invention. In one embodiment, "modified" primers are used in a PCR-like template and enzyme dependent synthesis. The primers may be modified by labelling with a capture moiety (e.g., biotin) and/or a detector moiety (e.g., enzyme). In the presence of a target sequence, the probe binds and is cleaved catalytically. After cleavage, the target sequences released intact to be bound by excess probe. Cleavage of the labelled probe signals the presence of the target sequence. In another approach, a nucleic acid amplification process involves cyclically synthesizing single-stranded RNA ("ssRNA"), SSDNA, and double-stranded DNA (dsDNA), which may be used in accordance with the present invention. The ssRNA is a first template for a first primer oligonucleotide, which is elongated by reverse transcriptase (RNA-dependent DNA polymerase). The RNA is then removed from the resulting DNA-RNA duplex by the action of ribonuclease H (RNase H, and RNase specific RNA in duplex with either DNA or RNA). The resultant ssDNA is a second template for a second primer, which also includes the sequences of an RNA polymerase promoter (exemplified by T7 RNA polymerase) 5' to its homology to the template. This primer is then extended by DNA polymerase (exemplified by the large "Klenow" fragment of *E coli* DNA polymerase I), resulting in a double-stranded ONA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

Method of Nucleic Acid Separation

It may be desirable to separate nucleic acid products from other materials, such as template and excess primer. In one embodiment, amplification products are separated by agarose, agarose acrylamide or polyacrylamide gel electrophoresis using standard methods (Sambrook et al., 1989, see infra). Separated amplification products may be cut out and eluted from the gel for further manipulation. Using low melting point agarose gels, the separated band may be removed by heating the gel, followed by extraction of the nucleic acid. Separation of nucleic acids may also be effected by chromatographic techniques known in the art. There are many kinds of chromatography which may be used in the practice of the present invention, including adsorption, partition, ion-exchange, hydroxylapatite, molecular sieve, reverse-phase, column, paper, thin-layer, and gas chromatography as well as HPLC. In certain embodiments, the amplification products are visualized. A typical visualization method involves staining of a gel with ethidium bromide and visualization of brands under UV light. Alternatively, if the amplification products are integrally labeled with radio- or fluorometrically-labeled nucleotides, the separated amplification products can be exposed to X-ray film or visualized with light exhibiting the appropriate excitatory spectra.

Alternatively, the presence of the polymorphic positions according to the methods of the invention can be determined by hybridization or lack of hybridization with a suitable nucleic acid probe specific for a polymorphic nucleic acid but not with the non-mutated nucleic acid.

By "hybridize" is meant a pair to form a double-stranded molecule between complementary polynucleotide sequences, or portions thereof, under various conditions of stringency. For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are all well known to those skilled in the art. Various levels of stringency are accomplished by combining these various conditions as needed. In a preferred embodiment, hybridization will occur at 30°C in 750 mM NaCl, 75 mM trisodium citrate, and 1% SDS. In a more preferred embodiment, hybridization will occur at 37°C in 500 mM NaCl, 50 mM trisodium citrate, 1% SDS, 35% formamide, and 100 μg/ml denatured salmon sperm DNA (ssDNA). In a most preferred embodiment, hybridization will occur at 42°C in 250 mM NaCl, 25 mM trisodium citrate, 1% SDS, 50% formamide, and 200 μg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred embodiment, wash steps will occur at 25°C in 30 mM NaCl, 3 mM trisodium citrate, and 0.1% SDS. In a more preferred embodiment, wash steps will occur at 42°C in 15 mM NaCl, 1.5 mM trisodium citrate, and 0.1% SDS. In a more preferred embodiment, wash steps will occur at 68°C in 15 mM NaCl, 1.5 mM trisodium citrate, and 0.1% SDS. Additional variations on these conditions will be readily apparent to those skilled in the art.

Hybridization techniques are well-known to those skilled in the art and are described, for example, in Benton and Davis (Science 196: 180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York, 1989.

Nucleic acid molecules useful for hybridization in the methods of the invention include any nucleic acid molecule which exhibits substantial identity so as to be able to specifically hybridize with the target nucleic acids. Polynucleotides having "substantial identity" to an endogenous sequence are typically capable of hybridizing with at least one strand of a double-stranded nucleic acid molecule. By "substantially identical" is meant a polypeptide or nucleic acid molecule exhibiting at least 50% identity to a reference amino acid sequence or nucleic molecule exhibiting at least 50% identity to a reference amino acid sequence or nucleic acid to the sequence used for comparison. Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical are similar sequences by assigning degrees of homology to various substitutions, deletions, and/or other modifications. Conservative substitutions typically include substitutions within the following groups: glycine, alanine, valine, isoleucine, leucine, aspartic acid, glutamic acid, asparagine, glutamine, serine, threonine, lysine, arginine, and phenylalanine, tyrosine. In an exemplary approach to determining the degree of identity, a BLAST program may be used with a probability score between e<"3> and e<"100> indicating a closely related sequence.

A detection system may be used to measure the absence, presence and amount of hybridization for all of the distinct sequences simultaneously. Preferably, a scanner is used to determine the levels and patterns of fluorescence.

The specific sequences which can be used for the detection of the polymorphisms are described in Table 5 below. Preferred SNPs are defined in the claims.

Table 5.

| SEQ ID № | GENE | dbSNP variant accession number | dbSNP sequence | Allele | Chrom | Position in GRCh37/ GRCh38 Chrom | Strand |
|---|---|---|---|---|---|---|---|
| 1 | F5 | rs4524) | CTTATGCTTAGCATGTTCTTGACTT**C**TGAA TTCTCCAGCACCAAGTGAAAG | Risk | 1 | 169511755/ 169542517 | (+) |
| 2 | | | CTTATGCTTAGCATGTTCTTGACTT**T**TGAA TTCTCCAGCACCAAGTGAAAG | Non-risk | | | |
| 3 | F5 | rs6025 | TGTAAGAGCAGATCCCTGGACAGGC**A**AG GAATACAGGTATTTTGTCCTTG | Risk | 1 | 169519049/ 169549811 | (-) |
| 4 | | | TGTAAGAGCAGATCCCTGGACAGGC**G**AG GAATACAGGTATTTTGTCCTTG | Non-risk | | | |
| 5 | SERPINE10 | rs2232698 | TTCAAACTTCGGATTCAGCCTGCTG**T**GAA AGATCTCCATGAGGCACGATGG | Risk | 14 | 94756669/ 94290332 | (-) |
| 6 | | | TTCAAACTTCGGATTCAGCCTGCTG**C**GAA AGATCTCCATGAGGCACGATGG | Non-risk | | | |
| 7 | SERPINE1 | rs2227631 | CCCCAAGTCCTAGCGGGCAGCTCGA**A**GA AGTGAAACTTACACGTTGGTCTC | Risk | 7 | 100769538/ 101126257 | (+) |
| 8 | | | CCCCAAGTCCTAGCGGGCAGCTCGA**G**GA AGTGAAACTTACACGTTGGTCTC | Non-risk | | | |
| 9 | LPL | rs268 | TGCAACAATCTGGGCTATGAGATCA**G**TAA AGTCAGAGCCAAAAGAAGCAG | Risk | 8 | 19813529/ 19956018 | (+) |
| 10 | | | TGCAACAATCTGGGCTATGAGATCA**A**TAA AGTCAGAGCCAAAAGAAGCAG | Non-risk | | | |

| SEQ ID № | GENE | dbSNP variant accession number | dbSNP sequence | Allele | Chrom | Position in GRCh37/ GRCh38 Chrom | Strand |
|---|---|---|---|---|---|---|---|
| 11 | HIVEP1 | rs169713 | TATTATTCGCACTCTACACAGCCAG**C**GTG GGGATTAATGGCATGATGTGCT | Risk | 6 | 11920517/ 11920284 | (+) |
| 12 | | | TATTATTCGCACTCTACACAGCCAG**T**GTG GGGATTAATGGCATGATGTGCT | Non-risk | | | |
| 13 | | rs11696364 | GCTACTAAAACTAAACACAGACTTA**A**CTG ATGACCAGGTATTTACCCAAGA | Risk | 20 | 1012026/ 1031383 | (+) |
| 14 | RSPO4 | | GCTACTAAAACTAAACACAGACTTA**C**CTG ATGACCAGGTATTTACCCAAGA | Non-risk | | | |
| 15 | APOA4 | rs5110 | GCACCTGCTCCTGCTGCTGCTCCTG**A**TGCT GTTCCTGCTGTTGCTCCAGCT | Risk | 11 | 116691634/ 116820918 | (+) |
| 16 | | | GCACCTGCTCCTGCTGCTGCTCCTG**C**TGCT GTTCCTGCTGTTGCTCCAGCT | Non-risk | | | |
| 17 | F13B | rs6003 | TTTGTACCCTGAAGCGCAACCATAA**T**GCA TGTTCTCTTGAATTTTATACAA | Risk | 1 | 197031021/19 7061891 | (+) |
| 18 | | | TTTGTACCCTGAAGCGCAACCATAA**C**GCA TGTTCTCTTGAATTTTATACAA | Non-risk | | | |
| NP = not pertinent | | | | | | | |

EP 4 134 450 A1

Method to establish the risk status

Another object of the present invention is the development of an algorithm - in close connection and relationship to the SNPs (and optionally further variables) to be determined - to estimate the risk of a patient suffering from cancer to develop and/or to being suffering a thromboembolic event or a recurrent thromboembolic event. The algorithm is shown as function 1.

Function 1

Estimating the risk of a thromboembolic event and/or recurrent event in a patient with cancer, either in the process of being diagnosed, just diagnosed, in whom chemotherapy is going to be initiated, or at any time from the diagnosis of cancer.

The individual estimation of the risk of a thromboembolic event is based on a logistic regression model. The aim of this model is to calculate the probability that a person has of presenting a thromboembolic event and/or a recurrent event according to his/her genetic, sociodemographic and clinical characteristics. To calculate this probability we use the following equation:

$$\text{Probability } (Y=1|X_1, ..., X_n) = 1 / 1 + \exp{(\beta_0 + \beta_1 X_1 + ... + \beta_n X_n + \beta_{f \cdot g} X_f \cdot X_g + ... \beta_{h \cdot i} X_h \cdot X_i)},$$

wherein:

- Probability $(1 | X_1,..., X_n)$ = probability of presenting a thromboembolic event in a particular cancer patient with concrete and measureable characteristics in a number of variables 1, ..., n. This probability could range between 0 and 1 (this is the probability considering all the variables included in the algorithm and it can be expressed as 0 - 1 or alternatively as a percentage from 0-100%. "0" means low probability and 1 (or 100%) means a high probability. The GRS score is one of the variables included and the GRS can range from 0 to 18, as described above);
- Exp = exponential natural base;
- $\beta_0$ = coefficient that defines the risk (the probability) of a thromboembolic event non-related with the variables 1 to n. This coefficient can take a value from $-\infty$ to $+\infty$ and is calculated as the natural logarithm of the incidence of thromboembolic events in the population:
- $\beta_1$ = regression coefficient that expresses the risk (higher or lower) to present a thromboembolic event associated with the value/presence of the predictor variable $X_1$. This coefficient can take a value from $-\infty$ to $+\infty$.
- $X_1$ = value taken by the predictor variable $X_1$ in an individual. The range of possible values depends on the variable;
- $\beta_n$ = regression coefficient that expresses the risk (higher or lower) to present a thromboembolic event associated with the value/presence of the predictor variable $X_n$. This coefficient can take a value from $-\infty$ to $+\infty$;
- $X_n$ = value taken by the predictor variable $X_n$ in an individual. The range of possible values depends on the variable.

In addition, the model includes the effect of the combination of some variables in terms of interaction or modification of the effect. That is, the effect size (regression coefficient) of a single variable ($X_f$) can be $\beta_f$ but if this variable is present in combination with another variable ($X_g$) the effect size may vary (increase or decrease). To consider the effect size of the variable $X_f$ it is therefore necessary to consider not only the $\beta_f$ but also a second regression coefficient $\beta_{f \cdot g}$ by adding the $\beta_f$ and the $\beta_{f \cdot g}$. Thus:

- $\beta_{f \cdot g}$ = regression coefficient that expresses the risk (higher or lower) to present a thromboembolic event associated with the combined presence of the predictor variables $X_f$ and $X_g$. This coefficient can take a value from $-\infty$ to $+\infty$;
- $X_f$ = value taken by the predictor variable $X_f$ in an individual. The range of possible values depends on the variable;
- $X_g$ = value taken by the predictor variable $X_g$ in an individual. The range of possible values depends on the variable;
- $\beta_{h \cdot i}$ = regression coefficient that expresses the risk (higher or lower) to present a thromboembolic event associated with the combined presence of the predictor variables $X_h$ and $X_i$ This coefficient can take a value from $-\infty$ to $+\infty$;

  - $X_h$ = value taken by the predictor variable $X_h$ in an individual. The range of possible values depends on the variable;
  - $X_i$ = value taken by the predictor variable $X_i$ in an individual. The range of possible values depends on the variable.

The variables included in the model and the regression coefficients of each of these variables are shown in Tables 2 and 4.

Surprisingly, the preferred combination of genetic variants (as described in Table 2), and even more preferred in combination with clinical variables included in the present invention (e.g. as set forth in Table 4), and further preferably using the function described in function 1, have proved to be capable to determine the risk of a patient with cancer to develop a thromboembolic disease or event or a recurrent thromboembolic disease or event, and to assist in the diagnosis of a thromboembolic disease or event or a recurrent thromboembolic disease or event, in a patient suffering from cancer with a higher accuracy that that obtained using the methods nowadays in use or published functions including genetic information. Surprisingly, the preferred combination of genetic variants, in particular in combination with clinical variables included in the present invention, and further preferably using the function described in function 1, have proved to be validated when tested in a different population to that used to develop it. This is particularly applicable to patients which have not yet undergone chemotherapy.

By the use of the functions described, a personalized risk for a patient with cancer is obtained for the development of a thromboembolic event or a recurrent thromboembolic event, in particular fatal- and non-fatal-myocardium infarction, stroke, transient ischemia attack, peripheral ateriopathy, deep vein thrombosis, pulmonary embolism or a combination thereof.

By the use of the above GRS, optionally in combination with further clinical risk factors, and optionally using the above described function, a personalized risk for a patient with cancer is obtained for the development of a thromboembolic event or a recurrent thromboembolic event, in particular fatal- and non-fatal-myocardium infarction, stroke, transient ischemia attack, peripheral ateriopathy, deep vein thrombosis, pulmonary embolism or a combination thereof.

Surprisingly, the VTE risk estimation can be performed at the same time the characterization of the cancer process is being performed by the physician, even before the consideration of a chemotherapy is established. This is very relevant as close to half of the VTE events in cancer patients are occuring even before chemotherapy is initiated.

*Definitions*

"Thromboembolic event" in the context of this application should be understood as the alteration of the hemostasis that leads to the development of a blood clot (thrombus) inside a vascular vessel (artery or vein). The thromboembolic event can also obstruct the vascular vessel completely and/or become detached and obstruct another vascular vessel.

"Thromboembolic event" is meant to include in the present application the following conditions among others: arterial thrombosis, fatal- and non-fatal myocardial infarction, stroke, transient ischemic attacks, cerebral venous thrombosis, peripheral arteriopathy, deep vein thrombosis, vein thrombosis and pulmonary embolism.

"Recurrent thromboembolic event" in the context of this application should be understood as a thromboembolic event in a subject who has already developed one or more thromboembolic events.

"Thromboembolic event" in the context of this application is used interchangeably with "thromboembolism".

"Thromboembolic event" in the context of this application is used interchangeably with "thrombosis".

"Thromboembolic event" in the context of this application is used interchangeably with "thromboembolic complication".

"Thrombophilia" in the context of this application should be understood as encompassing disorders of hemostasis that predispose to thrombosis. Included are heritable deficiencies of the natural anticoagulants antithrombin, protein C, and protein S, and un-common and common genetic variants in the genes encoding clotting factors as well as acquired thrombophilias, such as antiphospholipid antibodies. All these disorders and diseases are well known to the person skilled in the art.

The terms "disease" and "disorder" shall be interpreted in the context of this application interchangeably.

"Mutation" or "genetic variant" in the context of this application should be understood as the change of the sequence and/or structure of a gene, resulting in a variant form which may be transmitted to subsequent generations, caused by the alteration of single base units in the DNA, or the deletion, insertion, or rearrangement of larger sections of genes or chromosomes.

The terms "polymorphism" and "single nucleotide polymorphism" (SNP) are used herein interchangeably and relate to a genetic variant occurring when a single nucleotide in the genome or another shared sequence differs between members of species or between paired chromosomes in an individual. A SNP can also be designated as a mutation with low allele frequency greater than about 1% in a defined population. Single nucleotide polymorphisms according to the present application may fall within coding sequences of genes, non-coding regions of genes or the intronic regions between genes.

The term "sample", as used herein, refers to any sample from a biological source and includes, without limitation, cell cultures or extracts thereof, biopsied material obtained from a mammal or extracts thereof, and blood, saliva, urine, feces, semen, tears, or other body fluids or extracts thereof. A preferred sample in the present context is blood or saliva, even more preferred buccal cells.

"Predictive variables" in the context of this application should be understood as those described in Table 1 above.

"Sociodemographic and clinical characteristics" in the context of this application should be understood as age, gender, diabetes mellitus, smoking, family history of thromboembolic event, personal history of thromboembolic event, pregnancy, and body mass index.

### Example 1

The aim of this study was to evaluate the capability of a new genetic risk score and a new clinical-genetic algorithm (the so called "ONCOTHROMB") for the identification of cancer patients in an outpatient setting at risk of developing VTE in comparison to the standard risk assessment method. It is also the aim of this study to validate the new clinical-genetic algorithm in a different population to that used for the development.

**Methods**

*Study subjects*

The new ONCOTHROMB score was developed for predicting the risk of suffering a VTE within 6 months of a cancer diagnosis, using the data collected at diagnosis for 364 patients in the ONCOTHROMB 12-01 study (a single country, multicentre, observational, cohort study with an 18 month observation period involving data analysis at 6, 12 and 18 months; Clinicaltrial.gov identifier NCT03114618)[16]. To be included, patients have to be over 18 years of age, have a recent diagnosis of colorectal, oesophago-gastric, lung, or pancreatic cancer, an ECOG/WHO/Zubrod score of 0-2, be a candidate for systemic outpatient chemotherapy according to standard guidelines, and have no indication for mandatory thromboprophylactic therapy according to the attending oncologist.

The new score was validated using the clinical and genetic data of 263 patients included in the Vienna-CATS study[24]. These subjects, for whom samples for analysis of genetic variants were available, had the same primary cancers as those seen in the ONCOTHROMB 12-01 cohort. The Vienna-CATS study is an ongoing, prospective, single-centre, observational cohort study that involves blood collection and biobanking at study inclusion. Patients with a newly diagnosed cancer, or who experienced disease progression after complete or partial remission, were enrolled at a single academic tertiary centre in Vienna, Austria, between Oct 14, 2003 and March 26, 2014. Eligible patients for the Vienna-CATS study were those older than 18 years with a histologically confirmed diagnosis of cancer. At the time of inclusion, the exclusion criteria were an overt bacterial or viral infection within the past two weeks, venous or arterial thromboembolism within the past three months, and indication for continuous treatment with anticoagulants (vitamin K antagonists, direct oral anticoagulant or low-molecular-weight heparin). Patients were allowed treatment with aspirin, ticlopidine, or clopidogrel, and immobilized patients with low-molecular-weight heparin, to prevent thrombosis during any hospital stay that might be necessary. Other exclusion criteria were surgery or radiotherapy within the past two weeks of potential inclusion, and chemotherapy within the past three months, the aim being to exclude any transient effect of these interventions on haemostatic biomarkers.

Both the ONCOTHROMB 12-01 and Vienna-CATS studies were approved by the ethics committees of the participating hospitals. All patients provided written informed consent to be included.

The present report adheres to the Transparent Reporting of a Multivariable Prediction Model for Individual Prognosis or Diagnosis (TRIPOD) guidelines[25], and to the STARD2015 guidelines[26] for reporting studies on diagnostic accuracy.

*Genetic analysis*

Both the ONCOTHROMB 12-01 (development cohort) and Vienna-CATS (validation cohort) patients were genotyped for the genes shown in Table 6 using blood extracted at the time of diagnosis (ONCOTHROMB 12-01) or at any time during follow-up (Vienna-CATS) (in no case did early death prevent sample collection). Genotyping was performed using TaqMan genotyping assays and the EP1 Fluidigm (an efficient endpoint PCR system for high throughput SNP genotyping).

Table 6

| | |
|---|---|
| rs4524 | rs16861990 |
| rs6003 | rs1208134 |
| rs2070006 | rs2420371 |
| rs2066854 | rs10029715 |
| rs3136516 | rs78707713 |
| rs2227631 | rs2288904 |

(continued)

| | |
|---|---|
| rs268 | rs11696364 |
| rs9363864 | rs6034465 |
| rs9332695 | rs1883888 |
| rs3753305 | rs7025486 |
| rs4149755 | rs2192824 |
| rs1613662 | rs5110 |
| rs13146272 | rs150611042 |
| rs2227589 | rs2036914 |
| rs2289252 | rs1805010 |
| rs6050 | rs6025 |
| rs1800788 | rs1799963 |
| rs1063856 | rs1801020 |
| rs2232354 | rs5985 |
| rs2267667 | rs121909548 |
| rs3087505 | rs2232698 |
| rs17844078 | rs8176719 |
| rs1884841 | rs7853989 |
| rs45454293 | rs8176749 |
| rs1039084 | rs8176750 |
| rs169713 | |

## Diagnosis of VTE events

VTE events were verified by objective imaging methods. Deep vein thrombosis in the lower limbs was diagnosed by ultrasound or ascending venography. Pulmonary embolism was diagnosed by ventilation-perfusion lung scanning, pulmonary angiography, or spiral computed tomography. Intracranial venous thrombosis was diagnosed by magnetic resonance imaging.

## Development of the ONCOTHROMB risk score

The ONCOTHROMB score was developed in three steps:

1) Development of the genetic risk component.
Multivariate logistic regression was used to determine the weight of each genetic variable studied in the appearance of a VTE event within 6 months of diagnosis. A final genetic risk score was determined using the genetic variants associated with an increased risk of VTE in a multivariate model (p≤0.25).
2) Selection of clinical variables associated with the development of VTE.
Data on the clinical risk factors for cancer-associated VTE cited in the literature[27] were collected from all patients: primary tumour site, TNM stage, BMI, use of tobacco, age, sex, family (first degree) history of VTE, diabetes, hypertension, high blood cholesterol, previous surgery, platelet and leukocyte counts, immobilization, and the Khorana score. The risk of VTE according to the primary tumour site was categorized as in the Khorana score (low, high and very high)[28]. The risks associated with platelet and leukocyte counts were categorized using the same cutoffs used in the Khorana score[28]. Multivariate analysis was performed to determine which of these variables were associated with the occurrence of VTE within 6 months. Those associated with an increased risk of VTE (p≤0.25) were selected for developing the risk score.
3) Development of the clinical-genetic model.
The genetic risk score and the clinical variables selected were subjected to multivariate logistic regression analysis using an Akaike information criterion-based backward selection process[29].

***Comparing the ONCOTHROMB and Khorana scores***

The Khorana score and the proposed ONCOTHROMB score were calculated for each patient at the time of study inclusion, and their accuracy in terms of predicting observed VTE events compared. This was done by assessing the area under the receiver operating characteristic (ROC) curve (AUC; larger values indicate better discrimination)[30]. Standard measures of sensitivity, specificity, positive and negative predictive values (PPV and NPV), and positive and negative likelihood ratios (PLR and NLR)[31] were determined for specific cut-off points. For the Khorana score, the cut-off defining high risk was set at 3 or higher[3]. The ONCOTHROMB score cut-off point defining high risk was identified using the Youden J statistic as the point that maximizes the Youden index (defined as sensitivity+specificity-1)[32].
The Khorana and ONCOTHROMB risk scores were then compared in both the ONCOTHROMB 12-01 and Vienna-CATS populations.

Number of patients 'needed to treat'

To assess the ONCOTHROMB score in terms of its ability to help prevent VTE events, the number of patients 'needed to treat' (NNT) was determined under different conditions[33]. It was assumed that prophylactic medication would reduce cancer-associated VTE by 46%[34].

Proof-of-concept in other types of tumour

It is important to note that, for the validation of the ONCOTHROMB score, patients in the Vienna-CATS study with the same type of primary tumours as those in the ONCOTHROMB 12-01 cohort were selected. However, to provide a proof-of-concept that the ONCOTHROMB score has a wider scope, it was also validated in patients from the Vienna-CATS population with other types of primary tumours (n=397: breast cancer n=98 patients, lung n=123, colorectal n=60, pancreatic n=45, prostate n=22, kidney n=22, and stomach n=27) for whom the same type of clinical and genetic data as collected for the ONCOTHROMB 12-01 patients were available.

***Statistical analysis***

Continuous variables were described as means$\pm$SD, and categorical variables as proportions. Continuous variables were compared using the Student t-test or Mann-Whitney U test as required. Categorical variables were compared using the $X^2$ or Fisher Exact tests. The DeLong test was used to examine the difference between AUCs. All calculations were made using MedCalc Statistical Software v.18.11.3 (MedCalc Software bvba, Ostend, Belgium; https://www.medcalc.org; 2019).

**Results**

Characteristics of the ONCOTHROMB 12-01 patients

Table 7 shows the clinical and demographic characteristics, and Table 8 the distribution of genotypes, for the 364 ONCOTHROMB 12-01 patients. For each variable, the associated number and percentage of patients who experienced a VTE within 6 months of follow-up are shown. The overall incidence of VTE was 17.86%. Patients suffering from pancreatic cancer experienced VTEs at a significantly higher frequency (41.18%) than did those with other types of cancer (p<0.001). Patients with stage IV tumours, and patients with tumours in very high risk sites, also suffered significantly more VTEs.

Table 7

| | ONCOTHROMB | | |
|---|---|---|---|
| | **VTE (N=65)** | **NO-VTE (N=299)** | **p** |
| | | | |
| Gender, n (%) | | | |
| Female | 26 (40.0%) | 105 (35.1%) | 0.456 |
| Male | 39 (60.0%) | 194 (64.9%) | 0.456 |

(continued)

| | | | |
|---|---|---|---|
| Gender, n (%) | | | |
| Age, mean (sd) | 64.1 (11.3) | 64.4 (10.6) | 0.839 |
| Family history of VTE: | | | |
| No | 61 (93.8%) | 288 (96.3%) | 0.360 |
| Yes | 4 (6.15%) | 11 (3.68%) | 0.364 |
| BMI, n (%) | | | |
| <25 | 33 (50.8%) | 160 (53.5%) | 0.693 |
| 25-30 | 21 (32.3%) | 101 (33.8%) | 0.816 |
| >30 | 11 (16.9%) | 38 (12.7%) | 0.369 |
| Current Smoking, n (%) | | | |
| No | 44 (67.7%) | 235 (78.6%) | 0.060 |
| Yes | 21 (32.3%) | 64 (21.4%) | 0.060 |
| Pregnancy, n (%) | | | |
| No | 65 (100%) | 296 (99.0%) | 0.419 |
| Yes | 0 (0.00%) | 3 (1.00%) | 0.419 |
| Diabetes, n (%) | | | |
| No | 54 (83.1%) | 240 (80.3%) | 0.591 |
| Yes | 11 (16.9%) | 59 (19.7%) | 0.603 |
| Dislipidemia, n (%) | 27 (41.5%) | 99 (33.1%) | 0.25 |
| Hypertension, n (%) | 31 (47.7%) | 134 (44.8%) | 0.776 |
| TumorType, n (%): | | | |
| Colorectal | 20 (30.8%) | 132 (44.1%) | 0.049 |
| Pancreas | 28 (43.1%) | 40 (13.4%) | <0.0001 |
| Lung | 9 (13.8%) | 73 (24.4%) | 0.064 |
| Esophagus | 2 (3.08%) | 11 (3.68%) | 0.813 |
| Stomach | 6 (9.23%) | 43 (14.4%) | 0.269 |
| Stage: | | | |
| I+II | 4 (6.15%) | 61 (20.4%) | 0.006 |
| III | 14 (21.5%) | 117 (39.1%) | 0.007 |
| IV | 47 (72.3%) | 121 (40.5%) | <0.0001 |
| VTE risk Tumor Type (according to Khorana score), n (%) | | | |
| Low Risk | 22 (33.8%) | 143 (47.8%) | 0.04 |
| High Risk | 9 (13.8%) | 73 (24.4%) | 0.06 |
| Very High Risk | 34 (52.3%) | 83 (27.8%) | 0.0001 |
| Leukocyte >11×$10^9$/L, n (%) | | | 0.634 |
| No | 51 (78.5%) | 245 (81.9%) | 0.524 |
| Yes | 14 (21.5%) | 54 (18.1%) | 0.526 |
| Platelets >350×$10^9$/L, n (%) | | | 0.518 |
| No | 53 (81.5%) | 230 (76.9%) | 0.419 |

(continued)

| VTE risk Tumor Type (according to Khorana score), n (%) | | | |
|---|---|---|---|
| Yes | 12 (18.5%) | 69 (23.1%) | 0.419 |
| Haemoglobin >10 g/L, n (%) | | | 0.776 |
| No | 61 (93.8%) | 282 (94.3%) | 0.875 |
| Yes | 4 (6.15%) | 17 (5.69%) | 0.885 |
| Khorana Score ≥ 3 | 15 (23.1%) | 53 (17.7%) | 0.312 |
| D-Dimer ≥ 1.44 μg/mL | | | |
| | | | |
| | | CATS | |
| | **VTE (N=30)** | **NO-VTE (N=233)** | **p** |
| | | | |
| Gender, n (%) | | | |
| Female | 12 (40.0%) | 94 (40.34%) | 0.9715 |
| Male | 18 (60.0%) | 139 (59.66%) | 0.9715 |
| Age, mean (sd) | 64.27 (10.64) | 64.83 (10.71) | 0.9907 |
| Family history of VTE: | | | |
| No | ND | ND | |
| Yes | ND | ND | |
| BMI, n (%) | | | |
| <25 | 17 (56.67%) | 132 (56.66%) | 0.9992 |
| 25-30 | 10 (33.33%) | 67 (28.75%) | 0.6045 |
| >30 | 3 (10.0%) | 34 (14.59%) | 0.4969 |
| Current Smoking, n (%) | | | |
| No | 13 (43.33%) | 143 (61.38) | 0.0587 |
| Yes | 17 (56.67%) | 90 (38.62%) | 0.0587 |
| Pregnancy, n (%) | | | |
| No | 30 (100%) | 233 (100%) | |
| Yes | 0 | 0 | |
| Diabetes, n (%) | | | |
| No | 28 (93.33%) | 200 (85.83%) | 0.2560 |
| Yes | 2 (6.67%) | 33 (14.16%) | 0.2565 |
| Dislipidemia, n (%) | 3 (10.00%) | 27 (11.59%) | 0.7969 |
| Hypertension, n (%) | 14 (46.67%) | 99 (42.49%) | 0.6639 |
| TumorType, n (%): | | | |
| Colorectal | 6 (20%) | 54 (23.18%) | 0.6966 |
| Pancreas | 8 (26.67%) | 42 (18.03%) | 0.2573 |
| Lung | 12 (40.00%) | 114 (48.93%) | 0.3577 |
| Esophagus | 0 | 0 | |
| Stomach | 4 (13.33%) | 23 (9.87%) | 0.5575 |

(continued)

| Diabetes, n (%) | | | |
|---|---|---|---|
| Stage: | | | |
| I+II | 4 (13.33%) | 43 (18.45%) | 0.4916 |
| III | 5 (16.67%) | 45 (19.31%) | 0.7292 |
| IV | 21 (70%) | 145 (62.24&) | 0.4079 |
| VTE risk Tumor Type (according to Khorana score), n (%) | | | |
| Low Risk | 10 (33.33%) | 54 (23.18%) | 0.2236 |
| High Risk | 12 (40.00%) | 114 (48.93%) | 0.3577 |
| Very High Risk | 8 (26.67%) | 65 (27.89%) | 0.8885 |
| Leukocyte >11×10$^9$/L, n (%) | | | |
| No | 23 (76.67%) | 207 (88.84%) | 0.0587 |
| Yes | 7 (23.33%) | 26 (11.16%) | 0.0587 |
| Platelets >350×10$^9$/L, n (%) | | | |
| No | 23 (76.67%) | 187 (80.26%) | 0.6451 |
| Yes | 7 (23.33%) | 46 (19.74%) | 0.6451 |
| Haemoglobin >10 g/L, n (%) | | | |
| No | 28 (93.33%) | 218 (93.56%) | 0.9616 |
| Yes | 2 (6.67%) | 15 (6.44%) | 0.9616 |
| Khorana Score ≥ 3 | 6 (20%) | 25 (10.73%) | 0.1391 |
| D-Dimer ≥ 1.44 μg/mL | 17 (56.66%) | 53 (22.75%) | 0.0001 |

Table 8

| | Development population | | | Validation population | | |
|---|---|---|---|---|---|---|
| | **NoVTE (N=299)** | **VTE (N=65)** | **p** | **NoVTE (N=233)** | **VTE (N=30)** | **p** |
| rs4524: | | | 0.127 | | | 0.8853 |
| CC | 20 (6.69%) | 1 (1.54%) | | 26 (11.16%) | 3 (10.00%) | |
| CT | 110 (36.8%) | 20 (30.8%) | | 86 (36.91%) | 11 (36.67%) | |
| TT | 169 (56.5%) | 44 (67.7%) | | 121 (51.93%) | 16 (53.33%) | |
| rs2227631: | | | 0.256 | | | 0.3159 |
| AA | 88 (29.4%) | 22 (33.8%) | | 92 (39.48%) | 9 (30.00%) | |
| GA | 142 (47.5%) | 34 (52.3%) | | 103 (44.21%) | 16 (53.33%) | |
| GG | 69 (23.1%) | 9 (13.8%) | | 38 (16.31%) | 5 (16.67%) | |
| rs268: | | | 0.058 | | | 0.4183 |
| AA | 294 (98.3%) | 61 (93.8%) | | 228 (97.85%) | 30 (100.00%) | |
| GA | 5 (1.67%) | 4 (6.15%) | | 5 (2.15%) | 0 | |
| rs169713: | | | 0.08 | | | 0.6998 |
| CC | 17 (5.69%) | 2 (3.08%) | | 14 (6.01%) | 2 (6.66%) | |

(continued)

| | NoVTE (N=299) | VTE (N=65) | p | NoVTE (N=233) | VTE (N=30) | p |
|---|---|---|---|---|---|---|
| TC | 126 (42.1%) | 19 (29.2%) | | 94 (40.34%) | 11 (36.67%) | |
| TT | 156 (52.2%) | 44 (67.7%) | | 125 (53.65%) | 17 (56.67%) | |
| rs11696364: | | | 0.006 | | | 0.7195 |
| AA | 0 (0.00%) | 2 (3.08%) | | 1 (0.43%) | 0 | |
| CA | 31 (10.4%) | 12 (18.5%) | | 32 (13.73%) | 4 (13.33) | |
| CC | 268 (89.6%) | 51 (78.5%) | | 200 (85.84%) | 26 (86.67%) | |
| rs5110: | | | 0.091 | | | 0.4911 |
| GG | 257 (86.0%) | 50 (76.9%) | | 197 (84.55%) | 26 (86.66%) | |
| TG | 41 (13.7%) | 14 (21.5%) | | 37 (15.88%) | 3 (10%) | |
| TT | 1 (0.33%) | 1 (1.54%) | | 0 | 0 | |
| rs6025 | | | 0.637 | | | 0.3414 |
| GG | 314 (98.1) | 68 (95.8) | | 220 (94.42%) | 27 (90.00%) | |
| GA | 6 (1.9) | 3 (4.2) | | 13 (5.58%) | 3 (10.00%) | |
| rs2232698: | | | 0.205 | | | 0.6845 |
| CC | 293 (98.0%) | 62 (95.4%) | | 228 (97.85%) | 29 (96.67%) | |
| CT | 6 (2.01%) | 3 (4.62%) | | 5 (2.15%) | 1 (3.33%) | |
| rs6003: | | | 0.43 | | | 0.0376 |
| AA | 238 (80.1%) | 57 (87.7%) | | 209 (89.7%) | 23 (76.67%) | |
| GA | 57 (19.2%) | 8 (12.3%) | | 24 (10.3%) | 7 (23.33%) | |
| GG | 2 (0.67%) | 0 (0.00%) | | 0 | 0 (0.00%) | |

### *Development of the ONCOTHROMB score*

Table 9 shows the genetic and clinical markers that were associated with an increased risk of a VTE event ($p \leq 0.25$) in multivariate analysis, and thus selected for inclusion in the development of the ONCOTHROMB score. Although rs6025 (known as factor V Leiden) did not fulfil this inclusion criterion, it was included since there is strong evidence of its bearing on risk estimates for VTE in cancer patients[18—20,35—38].

## Table 9

|  | p value |
|---|---|
| GRS | 0.0001 |
| VTE  risk tumor | 0.008 |
| BMI > 25 | 0.131 |
| Stage | <0.0001 |

|  | p value |
|---|---|
| GRS |  |

| rs4524 | 0.106 |
|---|---|
| rs6025 | 0.403 |
| rs2232698 | 0.089 |
| rs2227631 | 0.143 |
| rs268 | 0.234 |
| rs169713 | 0.072 |
| rs11696364 | 0.015 |
| rs5110 | 0.117 |
| rs6003 | 0.1249 |

Accuracy of the ONCOTHROMB and Khorana scores

The ONCOTHROMB score showed significantly greater capacity than the Khorana score to distinguish between patients who would experience/not experience a VTE event (AUC 0.781 vs. 0.580; p<0.001)(Table 10). The sensitivity of the ONCOTHROMB score was also significantly better than that of the Khorana (81.54% vs. 22.54%; p<0.001), while the specificity of the Khorana score was higher (65.22% vs. 81.76%; p<0.0001). The PPV and NPV values of the ONCOTHROMB score were significantly higher than those of the Khorana score (PPV 33.758% vs. 23.63%; p=0.0025, and NPV 94.20% vs. 87.84%; p=0.0027) as were the likelihood ratios (LHR+ 2.34 vs. 1.42; p<0.0001, and LHR- 0.28 vs. 0.63; p<0.0001; for LHR+, higher values are better than lower, and for LHR-, lower values are better than higher) (Table 10). LR values are useful measures of diagnostic accuracy. LR+ indicates how many times more likely a positive test result occurs in subjects with the disease than in those without the disease. LR- indicates how many times more likely a negative test result occurs in subjects with the disease than in those without the disease. Unlike PPV and NPV, neither LR+ nor LR- depend on disease prevalence. LR values are directly linked to post-test probabilities[39].

Table 10

|  | Oncothromb | Khorana | p value |
|---|---|---|---|
|  |  |  |  |

(continued)

|  | Oncothromb | Khorana | p value |
|---|---|---|---|
| AUC | 0.781 (0.735-0.822) | 0.580 (0.51-0.65) | <0.0001 |
| p value | <0.0001 | 0.011 | |
| Sensitivity | 81.54 | 22.54 | <0.0001 |
| Specificity | 65.22 | 81.76 | 0.0001 |
| LHR+ | 2.34 | 1.42 | <0.0001 |
| LHR- | 0.28 | 0.63 | <0.0001 |
| PPV | 33.758 | 23.63 | 0.0025 |
| NPV | 94.20 | 87.84 | 0.0027 |

The NNT values were: a) 12, if all patients included in the study had been treated; b) 10, if only the patients with a Khorana score of ≥3 had been treated; and c) 6, if only patients with a high ONCOTHROMB score (with the cut-off set at the maximum Youden index value) had been treated. Under the criteria of drug effectiveness used (i.e., a reduction in cancer-associated VTE of 46%), the Khorana score would have helped prevent 19 of the 65 VTE detected, while the ONCOTHROMB score would have helped prevent 24. The administration of prophylactic treatment to all the patients included in the cohort would have helped prevent 30 VTE events with a non-significant higher risk of major bleeding events[34].

Validation of the ONCOTHROMB score

Table 7 shows the clinical and demographic characteristics, and Table 8 the distribution of genotypes, for the 263 patients of the Vienna-CATS cohort. For each variable, the associated number and percentage of patients who experienced a VTE within 6 months of follow-up are shown. The overall incidence of VTE was significantly lower than that observed in the ONCOTHROMB 12-01 population (11.40% vs. 17.86%, p=0.0134). Neither cancer type nor TNM stage were associated with a higher incidence of VTE in the Vienna-CATS cohort.

It is important to note that the proportions of patients with pancreatic and stomach cancer in both cohorts were similar, although colorectal and oesophagus cancers were more prevalent in the ONCOTHROMB 12-01 than in the Vienna-CATS cohort, and lung cancer was more common in the latter than in the former (Table 7). The incidence of VTE in patients with pancreatic cancer was higher in the ONCOTHROMB 12-01 than in the Vienna-CATS cohort (41.18% vs. 16.00%, p=0.0035), probably due to the more advanced tumour stage of the patients in the former. For all the other types of tumour, VTE incidence was similar in both cohorts.

Differences were seen between the cohorts in terms of the prevalence of genetic variants significantly associated with VTE (Table 8). In the ONCOTHROMB 12-01 cohort, rs11696364 was significantly more common in patients with VTE, while in the Vienna-CATS cohort, rs6003 was more common.

Accuracy of the ONCOTHROMB score when used with the Vienna-CATS cohort

In the Vienna-CATS cohort, the ONCOTHROMB score returned an AUC of 0.686 (0.626-0.742; *p<0.0001),* a sensitivity of 76.67%, and a specificity of 59.23%. Its PPV was 19.49%, NPV 95.17%, PLR 1.88, and NLR 0.39 (Table 11). The Khorana score failed to distinguish between patients who experienced/did not experience a VTE event (AUC 0.577; p=0.1627).

Table 11

| | Oncothromb | Khorana | p value |
|---|---|---|---|
| | | | |

(continued)

|  | | Oncothromb | Khorana | p value |
|---|---|---|---|---|
| AUC | | 0.686 (0.627-0.742) | 0.577 (0.514-0.637) | <0.0001 |
| p value | | <0.0001 | 0.1627 | |
| Sensitivity | | 76.67 | 20 | <0.0001 |
| Specificity | | 59.23 | 89.27 | <0.0001 |
| LHR+ | | 1.88 | 1.84 | 0.7224 |
| LHR- | | 0.39 | 0.896 | <0.0001 |
| PPV | | 19.42 | 19.35 | 0.8703 |
| NPV | | 95.17 | 89.65 | 0.0037 |

The sensitivity of the ONCOTHROMB score was significantly higher than that of the Khorana score (76.67% vs. 20.00%; p<0.0001), while the specificity of the Khorana score was higher (59.23% vs. 89.27%; p<0.0001). The NPV of the ONCOTHROMB score was significantly higher than that of the Khorana score (95.17% vs. 89.65%; *p*=0.0037), and the LHR- significantly better (0.394 vs. 0.896; p<0.0001). No differences were seen for PPV and LHR+ (PPV 19.49 vs. 19.35; p=0.8703, and LHR+ 1.88 vs. 1.84, p=0.7224).

The NNT values were: a) 19, if all patients included in the study had been treated; b) 10, if only the patients with a Khorana score of ≥3 had been treated; and c) 10, if only patients with a high risk ONCOTHROMB score (with the cut-off set at the maximum Youden index value) had been treated. Under the criteria of drug effectiveness used (i.e., a reduction in cancer-associated VTE of 46%), the Khorana score could have helped prevent 3 out of the 30 VTE detected, while the ONCOTHROMB score could have helped prevent 12. The administration of the prophylactic treatment to all the patients included in the cohort could have prevented 14 VTE with a non-significant higher risk of major bleeding events[34].

The difference in the AUC for the ONCOTHROMB score in the ONCOTHROMB 12-01 and Vienna-CATS populations was not significant (0.781 vs. 0.686; *p*=0.070).

In the ONCOTHROMB 12-01 cohort, the Khorana score identified 50 out of the 65 patients who suffered a VTE as being at low risk. The ONCOTHROMB score, however, reclassified 41 of those 50 patients as being at high risk for VTE. In the Vienna-CATS cohort, the Khorana score identified 24 out of the 30 patients who suffered a VTE as being at low risk for VTE. The ONCOTHROMB score, in contrast, reclassified 17 of those 24 as being at high risk for VTE.

It is important to note that for the validation of the ONCOTHROMB score, patients in the Vienna-CATS study were selected to have the same type of primary tumours as those in the ONCOTHROMB 12-01 cohort. However, as a proof-of-concept of the new score's wider use, patients from the Vienna-CATS population with other types of primary tumours were also included in the analysis. Similarly satisfactory results were obtained in this wider Vienna-CATS population (Table 12).

Table 12

|  | | Oncothromb |
|---|---|---|
| AUC | | 0.720 (0.673-0.762) |
| p value | | <0.0001 |
| Sensitivity | | 85.29 |
| Specificity | | 55.26 |
| LHR+ | | 1.91 |
| LHR- | | 0.27 |
| PPV | | 16.00 |
| NPV | | 95.76 |

Discussion

When deciding whether to use primary thromboprophylaxis in outpatients with cancer who are candidates for chemotherapy, a clinician needs to determine the risk of VTE and weigh the potential benefit against the possibility of bleeding. Despite the increasing awareness of cancer-associated VTE, and the increasing evidence of the benefits of thromboprophylaxis (coming from randomized clinical trials[40—45] and the latest meta-analyses[46—49]), its use is limited among outpatients. This is probably due to the sub-optimal capacity of the existing tools to predict the risk of experiencing a VTE event. In the most recent phase III randomized studies of primary thromboprophylaxis - the CASSINI and AVERT trials[40,45] - patients were selected based on their Khorana score. Despite recruiting only those with a Khorana score of ≥2, the incidence of VTE in the placebo arms was 10.2% and 8.8% in these trials respectively. The NNT for symptomatic VTE in these two trials combined was 40, only marginally more favourable than the NNT observed in low molecular weight heparin trials that involved the use of no risk assessment model[50] (e.g., in the SAVE ONCO trial[51] the NNT was 46). A risk assessment model with better predictive power might be used more often in clinical practice.

The present work presents a new predictive score, the ONCOTHROMB score, which shows significantly greater power to predict a VTE event than the Khorana score. In the development phase, nine genetic variants independently associated with VTE in out-patients with cancer were detected (Table 9). These were combined into a genetic risk score that showed a significant association with VTE. The ONCOTHROMB score combines this genetic risk score with three clinical variables also found to be independently associated with VTE in outpatients with cancer (Table 9). Along with tumour type and TNM stage, this GRS was among the three most important variables predictive of VTE risk. It should be noted that a family history of VTE was significantly associated with VTE in our earlier TiC-ONCO score study[16], but it was not associated with VTE in the development of this new score. This might be explained due to the fact that the earlier score involved four genetic variants while the new one involves nine. This extension of the genetic basis could have captured the information provide by family history.

From a clinical point of view, the ONCOTHROMB score identified patients at high risk significantly better than did the Khorana score (AUC 0.781 vs. 0.580; p<0.0001; Table 10). Among the patients identified at high risk by the ONCOTHROMB score, 34% eventually suffered a VTE event (Table 10); in comparison, 24% of the patients at high risk according to the Khorana score suffered a VTE (p=0.025). Another demonstration of the clinical usefulness of the ONCOTHROMB score is its capacity to amend the misclassifications made by the Khorana score; the ONCOTHROMB score properly classified as being at high risk 82% of those patients who developed a VTE - patients the Khorana score identified as being at low risk.

Following the accepted recommendations for developing and validating the new score[52,53], external validation was performed in an independent cohort - that of the well-known Vienna-CATS study. It is important to highlight that, in this validation cohort, the AUC for the ONCOTHROMB score was significantly higher than that for the Khorana score (0.686 vs. 0.577; p<0.0001) and not significantly different to that obtained in the development (ONCOTHROMB 12-01) cohort. Neither were differences seen in the sensitivity, specificity or NPV. However, the PPV was significantly lower than in the ONCOTHROMB 12-01 cohort (19.42 vs. 33.375; p=0.0001). Similarly, the AUC and the sensitivity for the Khorana score in the validation cohort were not significantly different to those obtained in the development cohort. However, the specificity of the Khorana score in the validation cohort was significantly higher (89.27 vs. 81.76; p<0.004).

It is important to note that, for the validation of the ONCOTHROMB score, patients in the Vienna-CATS study with the same type of primary tumours as those in the ONCOTHROMB 12-01 cohort were selected. However, as proof-of-concept, patients from the Vienna-CATS population with other types of primary tumours (for whom the same clinical and genetic data as collected from the ONCOTHROMB patients were available), were also analyzed, and similarly satisfactory results were obtained (Table 12).

The message for the clinical oncologist is that, from the time of diagnosis until 6 months later, the new ONCOTHROMB score for VTE risk in outpatients with cancer: 1) better identifies those at high risk of suffering VTE from those at low risk (the ONCOTHROMB score has a higher AUC value than the Khorana score); 2) identifies a larger number of patients likely to suffer a VTE (the ONCOTHROMB score is more sensitive than the Khorana score); 3) provides a more reliable classification of low risk (the NPV of the ONCOTHROMB score is higher than that of the Khorana score); and 4) the ONCOTHROMB score can amend most (71-82%) of the misclassifications made by the Khorana score in patients with VTE.

In summary, this study reports a validated, clinical-genetic risk score that is significantly better than the Khorana score at identifying outpatients with cancer at high risk of experiencing a VTE event. Patients identified as being at high risk by the ONCOTHROMB risk score would likely benefit from thromboprophylaxis despite the risk of haemorrhage. As the risk of cancer-associated VTE is high even at 6 months before cancer diagnosis, and the peak incidence is from 0 to 6 months post-diagnosis[2,3], it is recommended that the ONCOTHROMB score be calculated at the moment cancer is suspected[4,56]. This new score could improve the prediction, prevention and treatment of VTE in oncology outpatients, providing for more efficient and safer thromboprophylaxis in those at high risk. The results could change clinical practice and have an important impact in national health systems.

Also, the ONCOTHROMB score shows improved predictive and diagnostic value than the "TiC-Onco Score" described in WO2018215590 A1 (see Table 13).

Table 13

|  |  | Oncothromb score | TiC-Onco score |
|---|---|---|---|
|  |  |  |  |
| AUC |  | 0.781 (0.735-0.822) | 0.734 (0.673-0.794) |
| p-value* |  | p<0.0001 | p<0.001 |
| Sensitivity |  | 81.54 | 85.92 |
| Specificity |  | 65.22 | 49.06 |
| PPV |  | 33.758 | 27.23 |
| NPV |  | 94.20 | 94.01 |
| (*) p-values obtained when comparing Oncothromb score and TiC-Onco score vs Khorana score (the SoC) |  |  |  |

The GRS of the present Oncothromb test also shows improved predictive and diagnostic value compared to the GRS of the TiC-Onco Score (p-values of <0.0001 and 0.0049 respectively) independently of the clinical variables.

References of the example 1:

1. Noble S, Pasi J. Epidemiology and pathophysiology of cancer-associated thrombosis. Br J Cancer. 2010;102:S2-S9. doi:10.1038/sj.bjc.6605599

2. Blom JW, Doggen CJM, Osanto S, Rosendaal FR. Malignancies, prothrombotic mutations, and the risk of venous thrombosis. JAMA. 2005;293(6):715-722. doi: 10.1001/jama.293.6.715

3. Khorana AA, Dalal M, Lin J, Connolly GC. Incidence and predictors of venous thromboembolism (VTE) among ambulatory high-risk cancer patients undergoing chemotherapy in the United States. Cancer. 2013;119(3):648-655. doi: 10.1002/cncr.27772

4. Lyman GH, Bohlke K, Falanga A, American Society of Clinical Oncology. Venous thromboembolism prophylaxis and treatment in patients with cancer: American Society of Clinical Oncology clinical practice guideline update. J Oncol Pract. 2015;11(3):e442-4. doi:10.1200/JOP.2015.004473

5. Mu??oz Mart??n AJ, Font Puig C, Navarro Mart??n LM, Borrega Garc??a P, Mart??n Jim??nez M. Clinical guide SEOM on venous thromboembolism in cancer patients. Clin Transl Oncol. 2014;16(12):1079-1090. doi:10.1007/s12094-014-1238-y

6. Falanga A, Roila F, Mandala M. Management of venous thromboembolism (VTE) in cancer patients: ESMO Clinical Practice Guidelines clinical practice guidelines. Ann Oncol 22. 2011;22(Supplement 6):85-92. doi:10.1093/annonc/mdr392

7. Farge D, Frere C, Connors JM, et al. 2019 International Clinical Practice Guidelines for the Treatment and Prophylaxis of Venous Thromboembolism in Patients With Cancer. Lancet Oncol. 2019;2045(September). doi:10.1016/s1470-2045(19)30336-5

8. Key NS, Khorana AA, Kuderer NM, et al. Venous Thromboembolism Prophylaxis and Treatment in Patients With Cancer: ASCO Clinical Practice Guideline Update. J Clin Oncol. 2019. doi:10.1200/jco.19.01461

9. Wang T, Zwicker JI, Ay C, et al. The use of direct oral anticoagulants for primary thromboprophylaxis in ambulatory cancer patients: Guidance from the SSC of the ISTH. J Thromb Haemost. 2019. doi:10.1111/jth.14564

10. Khorana AA, Kuderer NM, Culakova E, Lyman GH, Francis CW. Development and validation of a predictive model for chemotherapy-associated thrombosis. 2015;111(10):4902-4908. doi:10.1182/blood-2007-10-116327.An

11. van Es N, Franke VF, Middeldorp S, Wilmink JW, Büller HR. The Khorana score for the prediction of venous thromboembolism in patients with pancreatic cancer. Thromb Res. 2017. doi:10.1016/j.thromres.2016.12.013

12. Tafur AJ, Caprini JA, Cote L, et al. Predictors of active cancer thromboembolic outcomes: RIETE experience of the Khorana score in cancer-associated thrombosis. Thromb Haemost. 2017. doi:10.1160/TH16-11-0840

13. Muñoz Martin AJ, Garcia Alfonso P, Rupérez Blanco AB, Perez Ramírez S, Blanco Codesido M, Martin Jiménez M. Incidence of venous thromboembolism (VTE) in ambulatory pancreatic cancer patients receiving chemotherapy and analysis of Khorana's predictive model. Clin Transl Oncol. 2014;16(10):927-930. doi:10.1007/s12094-014-1165-y

14. Pabinger I, van Es N, Heinze G, et al. A clinical prediction model for cancer-associated venous thromboembolism: a development and validation study in two independent prospective cohorts. Lancet Haematol. 2018;5(7):e289-e298. doi:10.1016/S2352-3026(18)30063-2

15. Gerotziafas GT, Taher A, Abdel-Razeq H, et al. A Predictive Score for Thrombosis Associated with Breast, Colorectal, Lung, or Ovarian Cancer: The Prospective COMPASS-Cancer-Associated Thrombosis Study. Oncologist. 2017:theoncologist.2016-0414. doi:10.1634/theoncologist.2016-0414

16. Muñoz Martin AJ, Ortega I, Font C, et al. Multivariable clinical-genetic risk model for predicting venous thromboembolic events in patients with cancer. Br J Cancer. 2018;(January): 1-6. doi:10.1038/s41416-018-0027-8

17. Cella CA, Di Minno G, Carlomagno C, et al. Preventing Venous Thromboembolism in Ambulatory Cancer Patients: The ONKOTEV Study. Oncologist. 2017;22(5):601-608. doi:10.1634/theoncologist.2016-0246

18. Gran O V., Brækkan SK, Hansen J-B. Prothrombotic genotypes and risk of venous thromboembolism in cancer. Thromb Res. 2018;164:S12-S18. doi:10.1016/j.thromres.2017.12.025

19. Pabinger I, Ay C, Dunkler D, et al. Factor V Leiden mutation increases the risk for venous thromboembolism in cancer patients - results from the Vienna Cancer And Thrombosis Study (CATS). J Thromb Haemost. 2015;13:17-22. doi:10.1111/jth.12778

20. Gran O V, Smith EN, Brækkan SK, et al. Joint effects of cancer and variants in the factor 5 gene on the risk of venous thromboembolism. Haematologica. 2016;101(9):1046-1053. doi:10.3324/haematol.2016.147405

21. Soria JM, Morange P-E, Vila J, et al. Multilocus genetic risk scores for venous thromboembolism risk assessment. J Am Heart Assoc. 2014;3(5):e001060. doi:10.1161/JAHA.114.001060

22. Hinds DA, Buil A, Ziemek D, et al. Genome-wide association analysis of self-reported events in 6135 individuals and 252 827 controls identifies 8 loci associated with thrombosis. Hum Mol Genet. 2016;25(9):1867-1874. doi:10.1093/hmg/ddw037

23. Tang W, Teichert M, Chasman DI, et al. A Genome-Wide Association Study for Venous Thromboembolism: The Extended Cohorts for Heart and Aging Research in Genomic Epidemiology (CHARGE) Consortium. Genet Epidemiol. 2013;37(5):512-521. doi:10.1002/gepi.21731

24. Ay C, Simanek R, Vormittag R, et al. High plasma levels of soluble P-selectin are predictive of venous thromboembolism in cancer patients: Results from the Vienna Cancer and Thrombosis Study (CATS). Blood. 2008. doi:10.1182/blood-2008-02-142422

25. Collins GS, Reitsma JB, Altman DG, Moons KGM. Transparent reporting of a multivariable prediction model for individual prognosis or diagnosis (TRIPOD): The TRIPOD Statement. Eur Urol. 2015;67(6):1142-1151. doi:10.1016/j.eururo.2014.11.025

26. Bossuyt PM, Reitsma JB, Bruns DE, et al. STARD 2015: An updated list of essential items for reporting diagnostic accuracy studies. Clin Chem. 2015;61(12):1446-1452. doi:10.1373/clinchem.2015.246280

27. Ay C, Pabinger I, Cohen AT. Cancer-associated venous thromboembolism: Burden, mechanisms, and management. Thromb Haemost. 2017;117(2):219-230. doi:10.1160/TH16-08-0615

28. Khorana AA, Kuderer NM, Culakova E, Lyman GH, Francis CW. Development and validation of a predictive model for chemotherapy-associated thrombosis. Blood. 2008;111(10):4902-4907.doi:10.1182/blood-2007-10-116327

29. Venables WN, Ripley BD. Modern Applied Statistics with S. Issues of Accuracy and Scale. 2002;(March):868. doi:10.1198/tech.2003.s33

30. Hanley J a., Hajian-Tilaki KO. Sampling variability of nonparametric estimates of the areas under receiver operating characteristic curves: an update. Acad Radiol. 1997;4(1):49-58. doi:10.1016/S1076-6332(97)80161-4

31. Attia J. Moving beyond sensitivity and specificity: using likelihood ratios to. Aust Prescr. 2003;26(5):111-113.

32. Schisterman EF, Perkins NJ, Liu A, Bondell H. Optimal cut-point and its corresponding Youden Index to discriminate individuals using pooled blood samples. Epidemiology. 2005;16(1):73-81. http://www.ncbi.nlm.nih.gov/pubmed/15613948. Accessed September 12, 2016.

33. Sackett DL, Haynes RB. Summarizing the effects of therapy: a new table and some more terms. ACP J Club. 1997;127(1):A15-6. citeulike-article-id:6063611%5Cn#.

34. Di Nisio M, Porreca E, Otten H-M, Rutjes AWS. Primary prophylaxis for venous thromboembolism in ambulatory cancer patients receiving chemotherapy. Cochrane database Syst Rev. 2014;8(8):CD008500. doi:10.1002/14651858.CD008500.pub3

35. Decousus H, Moulin N, Quenet S, et al. Thrombophilia and risk of venous thrombosis in patients with cancer. Thromb Res. 2007;120 Suppl:S51-61. doi:10.1016/S0049-3848(07) 70130-5

36. Heraudeau A, Delluc A, Le Henaff M, et al. Risk of venous thromboembolism in association with factor V leiden in cancer patients - The EDITH case-control study. Garcia de Frutos P, ed. PLoS One. 2018;13(5):e0194973. doi:10.1371/journal. pone.0194973

37. Kennedy M, Andreescu ACM, Greenblatt MS, et al. Factor V Leiden, prothrombin 20210A and the risk of venous thrombosis among cancer patients. Br J Haematol. 2005;128(3):386-388. doi:10.1111/j.1365-2141.2004.05327.x

38. Overview A, Montagnana M, Lippi G, Danese E. Hemostasis and Thrombosis. 2017;1646. doi:10.1007/978-1-4939-7196-1

39. Eusebi P. Diagnostic Accuracy Measures. Cerebrovasc Dis. 2013;36(4):267-272. doi:10.1159/000353863

40. Khorana AA, Soff GA, Kakkar AK, et al. Rivaroxaban for Thromboprophylaxis in High-Risk Ambulatory Patients with Cancer. N Engl J Med. 2019;380(8):720-728. doi:10.1056/NEJMoa1814630

41. Agnelli G, Gussoni G, Bianchini C, et al. Nadroparin for the prevention of thromboembolic events in ambulatory patients with metastatic or locally advanced solid cancer receiving chemotherapy: a randomised, placebo-controlled, double-blind study. Lancet Oncol. 2009;10(10):943-949. doi:10.1016/S1470-2045(09)70232-3

42. Maraveyas A, Waters J, Roy R, et al. Gemcitabine versus gemcitabine plus dalteparin thromboprophylaxis in pancreatic cancer. Eur J Cancer. 2012;48(9):1283-1292. doi:10.1 016/j.ejca.2011.10.017

43. Agnelli G, George DJ, Kakkar AK, et al. Semuloparin for thromboprophylaxis in patients receiving chemotherapy for cancer. N Engl J Med. 2012;366(7):601-609. doi:10.1056/NEJMoa1108898

44. Pelzer U, Opitz B, Deutschinoff G, et al. Efficacy of Prophylactic Low-Molecular Weight Heparin for Ambulatory Patients With Advanced Pancreatic Cancer: Outcomes From the CONKO-004 Trial. J Clin Oncol. 2015;33(18):2028-2034. doi:10.1200/JCO.2014.55.1481

45. Carrier M, Abou-Nassar K, Mallick R, et al. Apixaban to Prevent Venous Thromboembolism in Patients with Cancer. N Engl J Med. 2019;380(8):711-719. doi:10.1056/NEJMoa1814468

46. Becattini C, Verso M, Muñoz A, Agnelli G. Updated meta-analysis on prevention of venous thromboembolism in ambulatory cancer patients. Haematologica. June 2019:haematol.2019.221424. doi:10.3324/haematol.2019.221424

47. Ben-Aharon I, Stemmer SM, Leibovici L, Shpilberg O, Sulkes A, Gafter-Gvili A. Low molecular weight heparin (LMWH) for primary thrombo-prophylaxis in patients with solid malignancies - systematic review and meta-analysis. Acta Oncol. 2014;53(9):1230-1237. doi:10.3109/0284186X.2014.934397

48. Di Nisio M, Porreca E, Ferrante N, Otten H, Cuccurullo F, Rutjes AW. Primary prophylaxis for venous thromboembolism in ambulatory cancer patients receiving chemotherapy. Cochrane Database Syst Rev. 2012;2(12):10-13. doi:10.1002/14651858.CD008500.pub4.Copyright

49. Tun NM, Guevara E, Oo TH. Benefit and risk of primary thromboprophylaxis in ambulatory patients with advanced pancreatic cancer receiving chemotherapy: a systematic review and meta-analysis of randomized controlled trials. Blood Coagul Fibrinolysis. 2016;27(3):270-274. doi:10.1097/MBC.0000000000000413

50. Agnelli G. Direct Oral Anticoagulants for Thromboprophylaxis in Ambulatory Patients with Cancer. N Engl J Med. 2019;380(8):781-783. doi:10.1056/NEJMe1816060

51. Agnelli G, George DJ, Kakkar AK, et al. Semuloparin for Thromboprophylaxis in Patients Receiving Chemotherapy for Cancer. N Engl J Med. 2012;366(7):601-609. doi:10.1056/NEJMoa1108898

52. Altman DG, Royston P. What do we mean by validating a prognostic model? Stat Med. 2000.

53. McGinn TG, Guyatt GH, Wyer PC, Naylor CD, Stiell IG, Richardson WS. Users' guides to the medical literature XXII: How to use articles about clinical decision rules. J Am Med Assoc. 2000. doi:10.1001/jama.284.1.79

54. Cohen a. T, Imfeld S, Markham J, Granziera S. The use of aspirin for primary and secondary prevention in venous thromboembolism and other cardiovascular disorders. Thromb Res. 2015;135(2):217-225. doi:10.1016/j.thromres.2014.11.036

55. Azboy I, Barrack R, Thomas AM, Haddad FS, Parvizi J. Aspirin and the prevention of venous thromboembolism following total joint arthroplasty. Bone Joint J. 2017;99-B(11):1420-1430. doi:10.1302/0301-620X.99B11.BJJ-2017-0337.R2

56. Gran O V, Smith EN, Brækkan SK, et al. Joint effects of cancer and variants in the factor 5 gene on the risk of venous thromboembolism. Haematologica. 2016;101(9):1046-1053. doi:10.3324/haematol.2016.147405

**References**

1. Noble S, Pasi J. Epidemiology and pathophysiology of cancer-associated thrombosis. Br J Cancer. 2010;102:S2-S9. doi:10.1038/sj.bjc.6605599.

2. Blom JW, Doggen CJM, Osanto S, Rosendaal FR. Malignancies, prothrombotic mutations, and the risk of venous thrombosis. JAMA. 2005;293(6):715-722. doi:10.1001/jama.293.6.715.

3. Qureshi W, Ali Z, Amjad W, et al. Venous Thromboembolism in Cancer: An Update of Treatment and Prevention in the Era of Newer Anticoagulants. Front Cardiovasc Med. 2016;3:24. doi:10.3389/fcvm.2016.00024.

4. Khorana AA, Dalal M, Lin J, Connolly GC. Incidence and predictors of venous thromboembolism (VTE) among ambulatory high-risk cancer patients undergoing chemotherapy in the United States. Cancer. 2013;119(3):648-655.

doi:10.1002/cncr.27772.

5. Gran O V, Smith EN, Brækkan SK, et al. Joint effects of cancer and variants in the factor 5 gene on the risk of venous thromboembolism. Haematologica. 2016;101(9):1046-1053. doi:10.3324/haematol.2016.147405.

6. Lyman GH, Khorana AA, Falanga A, et al. American Society of Clinical Oncology Guideline: Recommendations for venous thromboembolism prophylaxis and treatment in patients with cancer. J Clin Oncol. 2007;25(34):5490-5505. doi:10.1200/JCO.2007.14.1283.

7. Muñoz Martin AJ, Font Puig C, Navarro Martin LM, Borrega Garcia P, Martin Jiménez M. Clinical guide SEOM on venous thromboembolism in cancer patients. Clin Transl Oncol. 2014;16(12):1079-1090. doi:10.1007/s12094-014-1238-y.

8. Lyman GH, Bohlke K, Falanga A, American Society of Clinical Oncology. Venous thromboembolism prophylaxis and treatment in patients with cancer: American Society of Clinical Oncology clinical practice guideline update. J Oncol Pract. 2015;11(3):e442-4. doi:10.1200/JOP.2015.004473.

9. Wun T, White RH. Epidemiology of cancer-related venous thromboembolism. Best Pract Res Clin Haematol. 2009;22(1):9-23. doi:10.1016/j.beha.2008.12.001.

10. Wun T, White RH. Venous thromboembolism in patients with acute leukemia, lymphoma, and multiple myeloma. Thromb Res. 2010;125:S96-S102. doi:10.1016/S0049-3848(10)70024-4.

11. Nowak-Göttl U, Wermes C, Junker R, et al. Prospective evaluation of the thrombotic risk in children with acute lymphoblastic leukemia carrying the MTHFR TT 677 genotype, the prothrombin G20210A variant, and further prothrombotic risk factors. Blood. 1999;93(5):1595-1599. http://www.ncbi.nlm.nih.gov/pubmed/10029588.

12. Chew HK, Wun T, Harvey D, Zhou H, White RH. Incidence of Venous Thromboembolism and Its Effect on Survival Among Patients With Common Cancers. Arch Intern Med. 2006;166(4):458. doi:10.1001/archinte.166.4.458.

13. Falanga A. The Incidence and Risk of Venous Thromboembolism Associated With Cancer and Nonsurgical Cancer Treatment. Cancer Invest. 2009;27(1):105-115. doi:10.1080/07357900802563028.

14. Agnelli G, Bolis G, Capussotti L, et al. A clinical outcome-based prospective study on venous thromboembolism after cancer surgery: the @RISTOS project. Ann Surg. 2006;243(1):89-95. http://www.ncbi.nlm.nih.gov/pubmed/16371741. Accessed November 9, 2016.

15. KHORANA AA, FRANCIS CW, CULAKOVA E, KUDERER NM, LYMAN GH. Thromboembolism is a leading cause of death in cancer patients receiving outpatient chemotherapy. J Thromb Haemost. 2007;5(3):632-634. doi:10.1111/j.1538-7836.2007.02374.x.

16. Khorana AA, Kuderer NM, Culakova E, Lyman GH, Francis CW. Development and validation of a predictive model for chemotherapy-associated thrombosis. Blood. 2008;111(10):4902-4907. doi:10.1182/blood-2007-10-116327.

17. Hutten BA, Prins MH, Gent M, Ginsberg J, Tijssen JG, Büller HR. Incidence of recurrent thromboembolic and bleeding complications among patients with venous thromboembolism in relation to both malignancy and achieved international normalized ratio: a retrospective analysis. J Clin Oncol. 2000;18(17):3078-3083. doi:10.1200/jco.2000.18.17.3078.

18. Khorana AA, Streiff MB, Farge D, et al. Venous Thromboembolism Prophylaxis and Treatment in Cancer: A Consensus Statement of Major Guidelines Panels and Call to Action. J Clin Oncol. 2009;27(29):4919-4926. doi:10.1200/JCO.2009.22.3214.

19. Ugarte Fornell G, Otero Candelera R, Ferrer Galván M, Morillo Guerrero R, Elias Hernández T, Jara Palomares L. [Predictive Khorana's model in patients with venous thromboembolic disease and cancer]. Med clínica. 2013;141(11):479-481. doi:10.1016/j.medcli.2013.04.031.

20. Mu??oz Mart??n AJ, Garc??a Alfonso P, Rup??rez Blanco AB, P??rez Ram??rez S, Blanco Codesido M, Mart??n Jim??nez M. Incidence of venous thromboembolism (VTE) in ambulatory pancreatic cancer patients receiving chemotherapy and analysis of Khorana's predictive model. Clin Transl Oncol. 2014;16(10):927-930. doi:10.1007/s12094-014-1165-y.

21. Srikanthan A, Tran B, Beausoleil M, et al. Large retroperitoneal lymphadenopathy as a predictor of venous thromboembolism in patients with disseminated germ cell tumors treated with chemotherapy. J Clin Oncol. 2015;33(6):582-587. doi:10.1200/JCO.2014.58.6537.

22. Falanga A, Russo L. Epidemiology, risk and outcomes of venous thromboembolism in cancer. Hamostaseologie. 2012;32(2):115-125. doi:10.5482/ha-1170.

23. Hanley J a., Hajian-Tilaki KO. Sampling variability of nonparametric estimates of the areas under receiver operating characteristic curves: an update. Acad Radiol. 1997;4(1):49-58. doi:10.1016/S1076-6332(97)80161-4.

24. Attia J. Moving beyond sensitivity and specificity: using likelihood ratios to. Aust Prescr. 2003;26(5):111-113.

25. R Development Core Team. R: a Language and Enviroment for Statistical Computing. Vienna, Austria. doi:Access October 1, 2015.

26. Sackett DL, Haynes RB. Summarizing the effects of therapy: a new table and some more terms. ACP J Club.

1997;127(1):A15-6. citeulike-article-id:6063611%5Cn#.

27. Di Nisio M, Porreca E, Otten H-M, Rutjes AWS. Primary prophylaxis for venous thromboembolism in ambulatory cancer patients receiving chemotherapy. Cochrane database Syst Rev. 2014;8(8):CD008500. doi:10.1002/14651858.CD008500.pub3.

**Claims**

1. A method for thromboembolic event risk assessment in a subject suffering from cancer comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of the risk of suffering a thromboembolic disease or event and/or a recurrent thromboembolic disease or event.

2. A method for the diagnosis of developing or suffering a thromboembolic disease or event or a recurrent thromboembolic event in a subject suffering from cancer comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of the risk of suffering a thromboembolic disease or event and/or a recurrent thromboembolic disease or event.

3. The method of claim 1 or 2, further comprising the steps of determining in the sample the presence or absence of at least one, two, three, four or preferably all of the following polymorphisms: rs6025, rs4524, rs2227631, rs169713, and rs5110, or respective SNPs in strong linkage disequilibrium with these variants, whereby said presence is indicative of the risk of suffering a thromboembolic disease or event and/or a recurrent thromboembolic disease or event.

4. The method of any one preceding claim, wherein the thromboembolic disease or event is selected from the group of fatal or non-fatal myocardial infarction, stroke, transient, ischemic attacks, peripheral arterial disease, vein thrombosis, deep vein thrombosis, pulmonary embolism or a combination thereof, preferably from venous thromboembolism, deep vein thrombosis and pulmonary embolism.

5. A method for identifying a subject suffering from cancer in need of anticoagulant and/or antithrombotic therapy or in need of prophylactic antithrombotic and/or anticoagulant therapy comprising the steps of determining in a sample isolated from said subject the presence or absence of each of the following polymorphisms: rs2232698, rs268, rs11696364, and rs6003, or respective SNPs in strong linkage disequilibrium with these variants, whereby said presence is indicative of being in need of early and aggressive antithrombotic and/or anticoagulant therapy or in need of prophylactic antithrombotic and/or anticoagulant therapy.

6. The method of claim 5, further comprising the steps of determining in the sample the presence or absence of at least one, two, three, four, or preferably all of the following polymorphisms: rs6025, rs4524, rs2227631, rs169713, and rs5110, or respective SNPs in strong linkage disequilibrium with these variants, whereby said presence is indicative of being in need of early and aggressive antithrombotic and/or anticoagulant therapy or in need of prophylactic antithrombotic and/or anticoagulant therapy.

7. The method of any one preceding claim, further comprising determining one or more risk factor(s) selected from the group consisting of body mass index (BMI), primary site of the tumor and tumor stage, optionally wherein each of body mass index (BMI), primary site of the tumor, and tumor stage are determined.

8. The method of any one preceding claim, wherein the method is performed during a procedure to identify whether the subject is suffering from cancer and/or during a procedure to characterize the TNM stage of the cancer; or wherein the method is performed at any time from the diagnosis of the cancer.

9. The method of any one preceding claim, wherein the subject suffering from cancer is treated in an 'in or out-patient' setting.

10. The method of any one preceding claim, wherein the sample is an oral tissue sample, scraping or wash or a biological fluid sample, preferably saliva, urine or blood, or buccal cells.

**11.** The method of any one preceding claim, wherein the presence or absence of the genetic variants is identified by allele-specific PCR or other molecular identification methods.

**12.** A method of determining the probability of an individual suffering from cancer of presenting a thromboembolic disease or event or a recurrent thromboembolic disease or event, based on the presence of 1 to P classical risk factors and 1 to J polymorphisms selected from the group of rs2232698, rs6025, rs4524, rs2227631, rs268, rs169713, rs11696364, rs5110, and/or rs6003, or respective SNPs in strong linkage disequilibrium with these variants, using the formula (function 1):

$$\text{Probability } (Y=1|X_1, ..., X_n) = 1/\, 1 + \exp\,(\beta_0 + \beta_1 X_1 + ... + \beta_n X_n + \beta_{f \cdot g}\, x_f \cdot X_g + .... \beta_{h \cdot i}\, x_h \cdot x_i),$$

wherein:

- Probability $(Y = 1 | X_1, ..., X_n)$ = probability of presenting a thrombosis in a particular cancer patient - with concrete and measurable characteristics in a number of variables 1, ...., n;
- Exp = exponential natural base;
- $\beta_0$ = coefficient that defines the risk (the probability) of thrombosis non related with the variables 1 to n;
- $\beta_1$ = regression coefficient that expresses the risk (higher or lower) to present thrombosis associated with the value/presence of the predictor variable X1;
- $X_1$ = value taken by the predictor variable X1 in an individual;
- $\beta_n$ = regression coefficient that expresses the risk (higher or lower) to present thrombosis associated with the value/presence of the predictor variable $X_n$;
- $X_n$ = value taken by the predictor variable $X_n$ in an individual;

and

- $\beta_{f \cdot g}$ = regression coefficient that expresses the risk (higher or lower) to present thrombosis associated with the combined presence of the predictor variables $X_f$ and $X_g$;
- $X_f$ = value taken by the predictor variable $X_f$ in an individual;
- $X_g$ = value taken by the predictor variable $X_g$ in an individual;
- $\beta_{h \cdot i}$ = regression coefficient that expresses the risk (higher or lower) to present thrombosis associated with the combined presence of the predictor variables $X_h$ and $X_i$;
- $X_h$ = value taken by the predictor varible $X_h$ in an individual; and
- $X_i$ = value taken by the predictor variable $X_i$ in an individual.

**13.** The method of claim 12, wherein the 1 to J polymorphisms comprise rs2232698, rs268, rs11696364, and rs6003, or respective SNPs in strong linkage disequilibrium with these variants; and preferably wherein the 1 to J polymorphisms further comprise at least one, two, three, four, or preferably all of rs6025, rs4524, rs2227631, rs169713, and rs5110, or respective SNPs in strong linkage disequilibrium with these variants.

**14.** The method of any one of claims 12 to 13, wherein the 1 to P classical risk factors are selected from the group consisting of BMI, primary site of the tumor and tumor stage, optionally wherein the 1 to P classical risk factors include each of BMI, primary site of the tumor and tumor stage.

**15.** The method of any one of the preceding claims, wherein the detection of the presence or absence of the polymorphisms is carried out at a separate location from the subject, and optionally wherein the risk assessment or the need for therapy is reported back to the subject and/or a clinician from that separate location.

**16.** The method of any one of the preceding claims, further comprising the steps of a) receiving, at a location separate from that of the subject, the sample isolated from the subject; and b) sending the results of the method from said separate location to the subject and/or a clinician.

**17.** A computer program or a computer-readable media containing means for carrying out the method of any one preceding claim.

**18.** A kit comprising reagents for detecting the identity of the nucleotides from the group of rs2232698, rs268, rs11696364, and rs6003, or respective SNPs in strong linkage disequilibrium with these variants.

**19.** The kit of claim 18, further comprising reagents for detecting the identity of the nucleotides from at least one, two, three, four, or preferably all of rs6025, rs4524, rs2227631, rs169713, and rs5110, or respective SNPs in strong linkage disequilibrium with these variants.

**20.** The kit of claim 18 or 19, wherein the reagents comprise primer pairs specific for the amplification of regions comprising the variants.

**21.** The method or kit according to any one preceding claim, wherein a strong linkage disequilibrium is one with an $r^2$ value of more than 0.7, more than 0.8, or more than 0.9, wherein $r^2$ is defined as follows:

$$r^2(p_a, p_b, p_{ab}) = \frac{(p_{ab} - p_a p_b)^2}{p_a(1 - p_a)p_b(1 - p_b)},$$

where $p_{ab}$ is the frequency of haplotypes having allele $\alpha$ at locus 1 and allele $b$ at locus 2

**22.** The method or kit according to claim 21, wherein the respective SNPs in strong linkage disequilibrium with the variants are selected from the SNPs in Table 3.

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2018/215590 A1 (GENDIAG EXE SL [ES]) 29 November 2018 (2018-11-29) * p. 7-8, para. 1-2; p. 24; p. 31; claims 10, 11, 13 * | 1-22 | INV. C12Q1/6883 C12Q1/6886 |
| X | Anonymous: "Direct Cycle Sequencing Kit Limited Use Label License: Research Use Only", Applied biosystems , 1 February 2011 (2011-02-01), XP055726710, Retrieved from the Internet: URL:https://tools.thermofisher.com/content /sfs/manuals/cms_091370.pdf [retrieved on 2020-09-01] * the whole document * | 18,19, 21,22 | |
| X | MATSUZAKI HAJIME ET AL: "GENOTYPING OVER 100,000 SNPS ON A PAIR OF OLIGONUCLEOTIDE ARRAYS", NATURE METHODS, NATURE PUBLISHING GROUP US, NEW YORK, vol. 1, no. 2, 1 November 2004 (2004-11-01), pages 109-111, XP009082656, ISSN: 1548-7091, DOI: 10.1038/NMETH718 * the whole document * | 18,19, 21,22 | TECHNICAL FIELDS SEARCHED (IPC) C12Q |
| Y | EP 1 566 449 A1 (MAX DELBRUECK CENTRUM [DE]) 24 August 2005 (2005-08-24) * claims 1, 3 * | 1-11,13, 15,16,20 | |

-/--

The present search report has been drawn up for all claims

| Place of search | Date of completion of the search | Examiner |
|---|---|---|
| Munich | 25 January 2022 | Ripaud, Leslie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................................
& : member of the same patent family, corresponding
document

EPO FORM 1503 03.82 (P04C01)

**page 1 of 2**

Europäisches
Patentamt

European
Patent Office

Office européen
des brevets

# EUROPEAN SEARCH REPORT

Application Number

EP 21 38 2760

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | GERMAIN MARINE ET AL: "Genetics of Venous Thrombosis: Insights from a New Genome Wide Association Study", PLOS ONE, vol. 6, no. 9, 27 September 2011 (2011-09-27), page e25581, XP055882783, DOI: 10.1371/journal.pone.0025581 Retrieved from the Internet: URL:https://www.ncbi.nlm.nih.gov/pmc/articles/PMC3181335/pdf/pone.0025581.pdf> * abstract; table 7 * ----- | 1-11,13, 15,16,20 | |
| Y | WO 2017/079551 A1 (SCRIPPS RESEARCH INST [US]) 11 May 2017 (2017-05-11) * para. 8 * ----- | 1-11,13, 15,16,20 | |
| X | MUÑOZ MARTÍN ANDRÉS J ET AL: "Multivariable clinical-genetic risk model for predicting venous thromboembolic events in patients with cancer", BRITISH JOURNAL OF CANCER, NATURE PUBLISHING GROUP UK, LONDON, vol. 118, no. 8, 28 March 2018 (2018-03-28), pages 1056-1061, XP036871158, ISSN: 0007-0920, DOI: 10.1038/S41416-018-0027-8 [retrieved on 2018-03-28] * the whole document * ----- | 1-22 | TECHNICAL FIELDS SEARCHED (IPC) |

The present search report has been drawn up for all claims

| Place of search | Date of completion of the search | Examiner |
|---|---|---|
| Munich | 25 January 2022 | Ripaud, Leslie |

EPO FORM 1503 03.82 (P04C01)

## ANNEX TO THE EUROPEAN SEARCH REPORT
## ON EUROPEAN PATENT APPLICATION NO.

EP 21 38 2760

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2022

| Patent document cited in search report | | Publication date | Patent family member(s) | | Publication date |
|---|---|---|---|---|---|
| WO 2018215590 | A1 | 29-11-2018 | EP | 3631016 A1 | 08-04-2020 |
| | | | WO | 2018215590 A1 | 29-11-2018 |
| EP 1566449 | A1 | 24-08-2005 | EP | 1566449 A1 | 24-08-2005 |
| | | | US | 2005196794 A1 | 08-09-2005 |
| WO 2017079551 | A1 | 11-05-2017 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018215590 A1
- US 6159693 A
- US 4683195 A
- US 4683202 A
- US 4800156 A
- US 4883750 A

### Non-patent literature cited in the description

- *Am J Surg,* 1970, vol. 120, 527-530
- **FARGE et al.** *Panel,* 2019, vol. 4
- **FARGE et al.** *Panel,* 2019, vol. 3
- **BENTON ; DAVIS.** *Science,* 1977, vol. 196 (180
- **GRUNSTEIN ; HOGNESS.** *Proc. Natl. Acad. Sci., USA,* vol. 72, 3961, , 1975
- **AUSUBEL et al.** Current Protocols in Molecular Biology. Wiley Interscience, 2001
- **BERGER ; KIMMEL.** Guide to Molecular Cloning Techniques. Academic Press, 1987
- **SAMBROOK et al.** Molecular Cloning: A Laboratory Manual. Cold Spring Harbor Laboratory Press, 1989
- **NOBLE S ; PASI J.** Epidemiology and pathophysiology of cancer-associated thrombosis. *Br J Cancer,* 2010, vol. 102, S2-S9
- **BLOM JW ; DOGGEN CJM ; OSANTO S ; ROSENDAAL FR.** Malignancies, prothrombotic mutations, and the risk of venous thrombosis. *JAMA,* 2005, vol. 293 (6), 715-722
- **KHORANA AA ; DALAL M ; LIN J ; CONNOLLY GC.** Incidence and predictors of venous thromboembolism (VTE) among ambulatory high-risk cancer patients undergoing chemotherapy in the United States. *Cancer,* 2013, vol. 119 (3), 648-655
- **LYMAN GH ; BOHLKE K ; FALANGA A ; AMERICAN SOCIETY OF CLINICAL ONCOLOGY.** Venous thromboembolism prophylaxis and treatment in patients with cancer: American Society of Clinical Oncology clinical practice guideline update. *J Oncol Pract,* 2015, vol. 11 (3), e442-4
- **MU??OZ MART??N AJ ; FONT PUIG C ; NAVARRO MART??N LM ; BORREGA GARC??A P ; MART??N JIM??NEZ M.** Clinical guide SEOM on venous thromboembolism in cancer patients. *Clin Transl Oncol,* 2014, vol. 16 (12), 1079-1090
- **FALANGA A ; ROILA F ; MANDALA M.** Management of venous thromboembolism (VTE) in cancer patients: ESMO Clinical Practice Guidelines clinical practice guidelines. *Ann Oncol 22,* 2011, vol. 22 (6), 85-92
- **FARGE D ; FRERE C ; CONNORS JM et al.** 2019 International Clinical Practice Guidelines for the Treatment and Prophylaxis of Venous Thromboembolism in Patients With Cancer. *Lancet Oncol,* September 2019, 2045
- **KEY NS ; KHORANA AA ; KUDERER NM et al.** Venous Thromboembolism Prophylaxis and Treatment in Patients With Cancer: ASCO Clinical Practice Guideline Update. *J Clin Onco,* 2019
- **WANG T ; ZWICKER JI ; AY C et al.** The use of direct oral anticoagulants for primary thromboprophylaxis in ambulatory cancer patients: Guidance from the SSC of the ISTH. *J Thromb Haemost,* 2019
- **KHORANA AA ; KUDERER NM ; CULAKOVA E ; LYMAN GH ; FRANCIS CW.** *Development and validation of a predictive model for chemotherapy-associated thrombosis,* 2015, vol. 111 (10), 4902-4908
- **VAN ES N ; FRANKE VF ; MIDDELDORP S ; WILMINK JW ; BÜLLER HR.** The Khorana score for the prediction of venous thromboembolism in patients with pancreatic cancer. *Thromb Res,* 2017
- **TAFUR AJ ; CAPRINI JA ; COTE L et al.** Predictors of active cancer thromboembolic outcomes: RIETE experience of the Khorana score in cancer-associated thrombosis. *Thromb Haemost,* 2017
- **MUÑOZ MARTIN AJ ; GARCIA ALFONSO P ; RUPÉREZ BLANCO AB ; PEREZ RAMÍREZ S ; BLANCO CODESIDO M ; MARTIN JIMÉNEZ M.** Incidence of venous thromboembolism (VTE) in ambulatory pancreatic cancer patients receiving chemotherapy and analysis of Khorana's predictive model. *Clin Transl Oncol,* 2014, vol. 16 (10), 927-930
- **PABINGER I ; VAN ES N ; HEINZE G et al.** A clinical prediction model for cancer-associated venous thromboembolism: a development and validation study in two independent prospective cohorts. *Lancet Haematol,* 2018, vol. 5 (7), e289-e298

- **GEROTZIAFAS GT ; TAHER A ; ABDEL-RAZEQ H et al.** A Predictive Score for Thrombosis Associated with Breast, Colorectal, Lung, or Ovarian Cancer: The Prospective COMPASS-Cancer-Associated Thrombosis Study. *Oncologist,* 2017
- **MUÑOZ MARTIN AJ ; ORTEGA I ; FONT C et al.** Multivariable clinical-genetic risk model for predicting venous thromboembolic events in patients with cancer. *Br J Cancer,* January 2018, 1-6
- **CELLA CA ; DI MINNO G ; CARLOMAGNO C et al.** Preventing Venous Thromboembolism in Ambulatory Cancer Patients: The ONKOTEV Study. *Oncologist,* 2017, vol. 22 (5), 601-608
- **GRAN O V. ; BRÆKKAN SK ; HANSEN J-B.** Prothrombotic genotypes and risk of venous thromboembolism in cancer. *Thromb Res,* 2018, vol. 164, S12-S18
- **PABINGER I ; AY C ; DUNKLER D et al.** Factor V Leiden mutation increases the risk for venous thromboembolism in cancer patients - results from the Vienna Cancer And Thrombosis Study (CATS). *J Thromb Haemost,* 2015, vol. 13, 17-22
- **GRAN O V ; SMITH EN ; BRÆKKAN SK et al.** Joint effects of cancer and variants in the factor 5 gene on the risk of venous thromboembolism. *Haematologica,* 2016, vol. 101 (9), 1046-1053
- **SORIA JM ; MORANGE P-E ; VILA J et al.** Multilocus genetic risk scores for venous thromboembolism risk assessment. *J Am Heart Assoc,* 2014, vol. 3 (5), e001060
- **HINDS DA ; BUIL A ; ZIEMEK D et al.** Genome-wide association analysis of self-reported events in 6135 individuals and 252 827 controls identifies 8 loci associated with thrombosis. *Hum Mol Genet,* 2016, vol. 25 (9), 1867-1874
- **TANG W ; TEICHERT M ; CHASMAN DI et al.** A Genome-Wide Association Study for Venous Thromboembolism: The Extended Cohorts for Heart and Aging Research in Genomic Epidemiology (CHARGE) Consortium. *Genet Epidemiol,* 2013, vol. 37 (5), 512-521
- **AY C ; SIMANEK R ; VORMITTAG R et al.** High plasma levels of soluble P-selectin are predictive of venous thromboembolism in cancer patients: Results from the Vienna Cancer and Thrombosis Study (CATS). *Blood,* 2008
- **COLLINS GS ; REITSMA JB ; ALTMAN DG ; MOONS KGM.** Transparent reporting of a multivariable prediction model for individual prognosis or diagnosis (TRIPOD): The TRIPOD Statement. *Eur Urol,* 2015, vol. 67 (6), 1142-1151
- **BOSSUYT PM ; REITSMA JB ; BRUNS DE et al.** STARD 2015: An updated list of essential items for reporting diagnostic accuracy studies. *Clin Chem,* 2015, vol. 61 (12), 1446-1452
- **AY C ; PABINGER I ; COHEN AT.** Cancer-associated venous thromboembolism: Burden, mechanisms, and management. *Thromb Haemost,* 2017, vol. 117 (2), 219-230
- **KHORANA AA ; KUDERER NM ; CULAKOVA E ; LYMAN GH ; FRANCIS CW.** Development and validation of a predictive model for chemotherapy-associated thrombosis. *Blood,* 2008, vol. 111 (10), 4902-4907
- **VENABLES WN ; RIPLEY BD.** Modern Applied Statistics with S. *Issues of Accuracy and Scale,* March 2002, 868
- **HANLEY J A. ; HAJIAN-TILAKI KO.** Sampling variability of nonparametric estimates of the areas under receiver operating characteristic curves: an update. *Acad Radiol,* 1997, vol. 4 (1), 49-58
- **ATTIA J.** Moving beyond sensitivity and specificity: using likelihood ratios to. *Aust Prescr,* 2003, vol. 26 (5), 111-113
- **SCHISTERMAN EF ; PERKINS NJ ; LIU A ; BONDELL H.** Optimal cut-point and its corresponding Youden Index to discriminate individuals using pooled blood samples. *Epidemiology,* 2005, vol. 16 (1), 73-81, http://www.ncbi.nlm.nih.gov/pubmed/15613948
- **SACKETT DL ; HAYNES RB.** Summarizing the effects of therapy: a new table and some more terms. *ACP J Club,* 1997, vol. 127 (1), A15-6
- **DI NISIO M ; PORRECA E ; OTTEN H-M ; RUTJES AWS.** Primary prophylaxis for venous thromboembolism in ambulatory cancer patients receiving chemotherapy. *Cochrane database Syst Rev,* 2014, vol. 8 (8), CD008500
- **DECOUSUS H ; MOULIN N ; QUENET S et al.** Thrombophilia and risk of venous thrombosis in patients with cancer. *Thromb Res,* 2007, vol. 120, S51-61
- **HERAUDEAU A ; DELLUC A ; LE HENAFF M et al.** Risk of venous thromboembolism in association with factor V leiden in cancer patients - The EDITH case-control study. *PLoS One,* 2018, vol. 13 (5), e0194973
- **KENNEDY M ; ANDREESCU ACM ; GREENBLATT MS et al.** Factor V Leiden, prothrombin 20210A and the risk of venous thrombosis among cancer patients. *Br J Haemato,* 2005, vol. 128 (3), 386-388
- **OVERVIEW A ; MONTAGNANA M ; LIPPI G ; DANESE E.** *Hemostasis and Thrombosis,* vol. 2017, 1646
- **EUSEBI P.** Diagnostic Accuracy Measures. *Cerebrovasc Dis,* 2013, vol. 36 (4), 267-272
- **KHORANA AA ; SOFF GA ; KAKKAR AK et al.** Rivaroxaban for Thromboprophylaxis in High-Risk Ambulatory Patients with Cancer. *N Engl J Med,* 2019, vol. 380 (8), 720-728

- **AGNELLI G ; GUSSONI G ; BIANCHINI C et al.** Nadroparin for the prevention of thromboembolic events in ambulatory patients with metastatic or locally advanced solid cancer receiving chemotherapy: a randomised, placebo-controlled, double-blind study. *Lancet Oncol,* 2009, vol. 10 (10), 943-949
- **MARAVEYAS A ; WATERS J ; ROY R et al.** Gemcitabine versus gemcitabine plus dalteparin thromboprophylaxis in pancreatic cancer. *Eur J Cancer,* 2012, vol. 48 (9), 1283-1292
- **AGNELLI G ; GEORGE DJ ; KAKKAR AK et al.** Semuloparin for thromboprophylaxis in patients receiving chemotherapy for cancer. *N Engl J Med,* 2012, vol. 366 (7), 601-609
- **PELZER U ; OPITZ B ; DEUTSCHINOFF G et al.** Efficacy of Prophylactic Low-Molecular Weight Heparin for Ambulatory Patients With Advanced Pancreatic Cancer: Outcomes From the CONKO-004 Trial. *J Clin Oncol,* 2015, vol. 33 (18), 2028-2034
- **CARRIER M ; ABOU-NASSAR K ; MALLICK R et al.** Apixaban to Prevent Venous Thromboembolism in Patients with Cancer. *N Engl J Med,* 2019, vol. 380 (8), 711-719
- **BECATTINI C ; VERSO M ; MUÑOZ A ; AGNELLI G.** Updated meta-analysis on prevention of venous thromboembolism in ambulatory cancer patients. *Haematologica,* June 2019
- **BEN-AHARON I ; STEMMER SM ; LEIBOVICI L ; SHPILBERG O ; SULKES A ; GAFTER-GVILI A.** Low molecular weight heparin (LMWH) for primary thrombo-prophylaxis in patients with solid malignancies - systematic review and meta-analysis. *Acta Oncol,* 2014, vol. 53 (9), 1230-1237
- **DI NISIO M ; PORRECA E ; FERRANTE N ; OTTEN H ; CUCCURULLO F ; RUTJES AW.** Primary prophylaxis for venous thromboembolism in ambulatory cancer patients receiving chemotherapy. *Cochrane Database Syst Rev,* 2012, vol. 2 (12), 10-13
- **TUN NM ; GUEVARA E ; OO TH.** Benefit and risk of primary thromboprophylaxis in ambulatory patients with advanced pancreatic cancer receiving chemotherapy: a systematic review and meta-analysis of randomized controlled trials. *Blood Coagul Fibrinolysis,* 2016, vol. 27 (3), 270-274
- **AGNELLI G.** Direct Oral Anticoagulants for Thromboprophylaxis in Ambulatory Patients with Cancer. *N Engl J Med,* 2019, vol. 380 (8), 781-783
- **AGNELLI G ; GEORGE DJ ; KAKKAR AK et al.** Semuloparin for Thromboprophylaxis in Patients Receiving Chemotherapy for Cancer. *N Engl J Med,* 2012, vol. 366 (7), 601-609
- **ALTMAN DG ; ROYSTON P.** What do we mean by validating a prognostic model?. *Stat Med,* 2000
- **MCGINN TG ; GUYATT GH ; WYER PC ; NAYLOR CD.** Stiell IG, Richardson WS. Users' guides to the medical literature XXII: How to use articles about clinical decision rules. *J Am Med Assoc,* 2000
- **COHEN A. T ; IMFELD S ; MARKHAM J ; GRANZIERA S.** The use of aspirin for primary and secondary prevention in venous thromboembolism and other cardiovascular disorders. *Thromb Res,* 2015, vol. 135 (2), 217-225
- **AZBOY I ; BARRACK R ; THOMAS AM ; HADDAD FS ; PARVIZI J.** Aspirin and the prevention of venous thromboembolism following total joint arthroplasty. *Bone Joint J,* 2017, vol. 99-B (11), 1420-1430
- **NOBLE S ; PASI J.** Epidemiology and pathophysiology of cancer-associated thrombosis. *Br J Cancer,* 2010, vol. 102, S2-S9
- **QURESHI W ; ALI Z ; AMJAD W et al.** Venous Thromboembolism in Cancer: An Update of Treatment and Prevention in the Era of Newer Anticoagulants. *Front Cardiovasc Med,* 2016, vol. 3, 24
- **LYMAN GH ; KHORANA AA ; FALANGA A et al.** American Society of Clinical Oncology Guideline: Recommendations for venous thromboembolism prophylaxis and treatment in patients with cancer. *J Clin Oncol,* 2007, vol. 25 (34), 5490-5505
- **MUÑOZ MARTIN AJ ; FONT PUIG C ; NAVARRO MARTIN LM ; BORREGA GARCIA P ; MARTIN JIMÉNEZ M.** Clinical guide SEOM on venous thromboembolism in cancer patients. *Clin Transl Oncol,* 2014, vol. 16 (12), 1079-1090
- **LYMAN GH ; BOHLKE K ; FALANGA A.** American Society of Clinical Oncology. Venous thromboembolism prophylaxis and treatment in patients with cancer: American Society of Clinical Oncology clinical practice guideline update. *J Oncol Pract,* 2015, vol. 11 (3), e442-4
- **WUN T ; WHITE RH.** Epidemiology of cancer-related venous thromboembolism. *Best Pract Res Clin Haematol,* 2009, vol. 22 (1), 9-23
- **WUN T ; WHITE RH.** Venous thromboembolism in patients with acute leukemia, lymphoma, and multiple myeloma. *Thromb Res,* 2010, vol. 125, S96-S102
- **NOWAK-GÖTTL U ; WERMES C ; JUNKER R et al.** Prospective evaluation of the thrombotic risk in children with acute lymphoblastic leukemia carrying the MTHFR TT 677 genotype, the prothrombin G20210A variant, and further prothrombotic risk factors. *Blood,* 1999, vol. 93 (5), 1595-1599, http://www.ncbi.nlm.nih.gov/pubmed/10029588
- **CHEW HK ; WUN T ; HARVEY D ; ZHOU H ; WHITE RH.** Incidence of Venous Thromboembolism and Its Effect on Survival Among Patients With Common Cancers. *Arch Intern Med,* 2006, vol. 166 (4), 458

- **FALANGA A.** The Incidence and Risk of Venous Thromboembolism Associated With Cancer and Nonsurgical Cancer Treatment. *Cancer Invest,* 2009, vol. 27 (1), 105-115
- **AGNELLI G ; BOLIS G ; CAPUSSOTTI L et al.** A clinical outcome-based prospective study on venous thromboembolism after cancer surgery: the @RIS-TOS project. *Ann Surg,* 09 November 2016, vol. 243 (1), 89-95, http://www.ncbi.nlm.nih.gov/pubmed/16371741
- **KHORANA AA ; FRANCIS CW ; CULAKOVA E ; KUDERER NM ; LYMAN GH.** Thromboembolism is a leading cause of death in cancer patients receiving outpatient chemotherapy. *J Thromb Haemost,* 2007, vol. 5 (3), 632-634
- **HUTTEN BA ; PRINS MH ; GENT M ; GINSBERG J ; TIJSSEN JG ; BÜLLER HR.** Incidence of recurrent thromboembolic and bleeding complications among patients with venous thromboembolism in relation to both malignancy and achieved international normalized ratio: a retrospective analysis. *J Clin Oncol,* 2000, vol. 18 (17), 3078-3083
- **KHORANA AA ; STREIFF MB ; FARGE D et al.** Venous Thromboembolism Prophylaxis and Treatment in Cancer: A Consensus Statement of Major Guidelines Panels and Call to Action. *J Clin Oncol,* 2009, vol. 27 (29), 4919-4926
- **UGARTE FORNELL G ; OTERO CANDELERA R ; FERRER GALVÁN M ; MORILLO GUERRERO R ; ELIAS HERNÁNDEZ T ; JARA PALOMARES L.** Predictive Khorana's model in patients with venous thromboembolic disease and cancer. *Med clínica,* 2013, vol. 141 (11), 479-481
- **MU??OZ MART??N AJ ; GARC??A ALFONSO P ; RUP??REZ BLANCO AB ; P??REZ RAM??REZ S ; BLANCO CODESIDO M ; MART??N JIM??NEZ M.** Incidence of venous thromboembolism (VTE) in ambulatory pancreatic cancer patients receiving chemotherapy and analysis of Khorana's predictive model. *Clin Transl Oncol,* 2014, vol. 16 (10), 927-930
- **SRIKANTHAN A ; TRAN B ; BEAUSOLEIL M et al.** Large retroperitoneal lymphadenopathy as a predictor of venous thromboembolism in patients with disseminated germ cell tumors treated with chemotherapy. *J Clin Oncol,* 2015, vol. 33 (6), 582-587
- **FALANGA A ; RUSSO L.** Epidemiology, risk and outcomes of venous thromboembolism in cancer. *Hamostaseologie,* 2012, vol. 32 (2), 115-125
- *R Development Core Team. R: a Language and Enviroment for Statistical Computing. Vienna, Austria,* 01 October 2015